# EUROPÄISCHE PATENTSCHRIFT

(11) **EP 1 120 411 B1**
(45) Veröffentlichungstag und Bekanntmachung des Hinweises auf die Patenterteilung: **22.09.2004**
(21) Anmeldenummer: 01110136.7
(22) Anmeldetag: 04.12.1995
(51) Int. Cl.: C07D 249/12, A01N 43/653, C07D 413/04, C07D 405/04

(54) **4-Aryl-1,2,4-Triazolidindionderivate und ihre Verwendung als Herbizide**
4-Aryl-1,2,4-triazolidinedione derivatives and their use as herbicides
Dérivés de 4-aryl-1,2,4-triazolidinedione et leur utilisation comme herbicides

(30) Priorität: 15.12.1994 DE 4444741; 24.08.1995 DE 19531152
(43) Veröffentlichungstag der Anmeldung: 01.08.2001
(62) Teilanmeldung aus: 95942066.2
(73) Patentinhaber: Bayer CropScience AG, 40789 Monheim (DE); Bayer CropScience K.K., Tokyo (JP)
(72) Erfinder: Linker, Karl-Heinz, 51377 Leverkusen (DE); Findeisen, Kurt, Prof. Dr., 51375 Leverkusen (DE); Schallner, Otto, Dr., 40789 Monheim (DE); Lender, Andreas, Dr., 42327 Wuppertal (DE); Santel, Hans-Joachim, Dr., Leawood, KS 66209 (US); Dollinger, Markus, Dr., 51381 Leverkusen (DE); Yanagi, Akihiko, Oyama-shi, Tochigi (JP); Goto, Toshio, Kokubunji-machi, Tochigi (JP)
(74) Vertreter: Krieg, Robert Alexander, Dr.

(56) Entgegenhaltungen:
- EP-A- 0 011 693
- GB-A- 2 041 353
- GB-A- 2 055 826
- GB-A- 2 063 855
- US-A- 5 108 486
- CHEMICAL ABSTRACTS, vol. 89, no. 3, 17. Juli 1978 (1978-07-17) Columbus, Ohio, US; abstract no. 24319z, Seite 653; XP002168944 & JP 53 018571 A (MITSUBISHI CHEMICAL INDUSTRIES CO., LTD.) 20. Februar 1978 (1978-02-20)

## Beschreibung

Die Erfindung betrifft neue substituierte N-Aryl-Stickstoffheterocyclen, Verfahren zu ihrer Herstellung und ihre Verwendung als Herbizide.

Es ist bekannt, daß bestimmte N-Aryl-Stickstoffheterocyclen herbizide Eigenschaften aufweisen (vgl. EP 11693, DE 2952685, DE 3026739, US 4276420, US 4326878, WO 94/14817, EP 75267 und EP 210137).

In der GB-A-2055826 werden Triazolidin-3,5-dithion-Derivate mit herbiziden Eigenschaften genannt, die einen einfach oder zweifach substituierten Phenylrest besitzen können.

In der GB-A-2041353 und der GB-A-2063855 werden 1,2,4-trisubstituierte Triazolidin-3-on-5-thion-Derivate mit herbiziden Eigenschaften genannt, die einen einfach oder zweifach substituierten Phenylrest besitzen können.

In der EP-A-0011693 werden N-(Trifluormethansulfonylaminophenyl)-substituierte N-Heterocyclen mit herbiziden Eigenschaften genannt. Darunter befinden sich Triazolidin-3,5-dion-Derivate, die jedoch am Phenylrest ein im Vergleich zu den erfindungsgemäßen Verbindungen unterschiedliches Substitutionsmuster aufweisen.

In der US-A-5108486 werden substituierte 1,2,4-Triazol-5(1H)-thione und -one mit herbiziden Eigenschaften genannt.

In Chemicals Abstracts 89, 1978, 24319z wird 4-(3,5-Dichlor-4-fluorphenyl)urazol mit fungiziden Eigenschaften genannt.

Die aus den angegebenen Patentanmeldungen bekannten Verbindungen haben jedoch keine nennenswerte Bedeutung erlangt.

Es wurden nun die neuen substituierten N-Aryl-Stickstoffheterocyclen der allgemeinen Formel (I) gefunden, 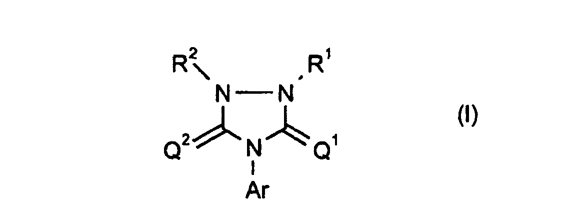 in welcher
- Q¹: für Sauerstoff oder Schwefel steht,
- Q²: für Sauerstoff oder Schwefel steht,
- R¹: für gegebenenfalls durch Fluor oder Chlor substituiertes C₁-C₆-Alkyl steht,
- R²: für gegebenenfalls durch Fluor oder Chlor substituiertes C₁-C₆-Alkyl steht, und
- Ar: für die nachstehend definierte substituierte, monocyclische Aryl-Gruppierung steht, 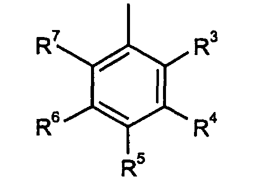 worin
R³ für Fluor, Chlor oder Brom steht,
R⁴ für Wasserstoff, Fluor, Chlor oder Brom steht,
R⁵ für Cyano, Carboxy, Chlorcarbonyl, Carbamoyl, Thiocarbamoyl, Hydroxy, Fluor, Chlor, Brom oder für jeweils gegebenenfalls durch Fluor und/oder Chlor substituiertes Alkyl, Alkoxy oder Alkoxycarbonyl mit jeweils bis zu 4 Kohlenstoffatomen steht,
R⁶ für die nachstehende Gruppierung steht,

-A¹-A²-A³

in welcher
A¹ für eine Einfachbindung oder für Sauerstoff, Schwefel, -SO-, -SO₂-, -CO- oder die Gruppierung -N-A⁴- steht, worin A⁴ für Wasserstoff, Hydroxy, C₁-C₄-Alkyl, C₃-C₄-Alkenyl, C₃-C₄-Alkinyl, C₁-C₄-Alkoxy, Phenyl, C₁-C₄-Alkyl-carbonyl, Phenylcarbonyl, C₁-C₄-Alkyl-sulfonyl oder Phenylsulfonyl steht,
A¹ weiterhin für jeweils gegebenenfalls durch Fluor, Chlor oder Brom substituiertes C₁-C₆-Alkandiyl, C₂-C₆-Alkendiyl, C₂-C₆-Azaalkendiyl, C₂-C₆-Alkindiyl, C₃-C₆-Cycloalkandiyl, C₃-C₆-Cycloalkendiyl oder Phenylen steht,
A² für eine Einfachbindung oder für Sauerstoff, Schwefel, -SO-, -SO₂-, -CO- oder die Gruppierung -N-A⁴- steht, worin A⁴ für Wasserstoff, Hydroxy, C₁-C₄-Alkyl, C₁-C₄-Alkoxy, Phenyl, C₁-C₄-Alkylsulfonyl oder Phenylsulfonyl steht,
A² weiterhin für jeweils gegebenenfalls durch Fluor, Chlor oder Brom substituiertes C₁-C₆-Alkandiyl, C₂-C₆-Alkendiyl, C₂-C₆-Azaalkendiyl, C₂-C₆-Alkindiyl, C₃-C₆-Cycloalkandiyl, C₃-C₆-Cycloalkendiyl oder Phenylen steht,
A³ für Wasserstoff steht, mit der Maßgabe, daß in diesem Fall A¹ und/oder A² nicht für eine Einfachbindung stehen,
A³ weiterhin für Hydroxy, Mercapto, Amino, Cyano, Isocyano, Thiocyanato, Nitro, Carboxy, Carbamoyl, Thiocarbamoyl, Sulfo, Chlorsulfonyl, Fluor, Chlor, Brom steht,
A³ weiterhin für jeweils gegebenenfalls durch Fluor, Chlor oder C₁-C₄-Alkoxy substituiertes Alkyl, Alkoxy, Alkylthio, Alkylsulfinyl, Alkylsulfonyl, Alkylamino, Dialkylamino, Alkoxycarbonyl oder Dialkoxy(thio)phosphoryl mit jeweils 1 bis 6 Kohlenstoffatomen in den Alkylgruppen steht,
A³ weiterhin für jeweils gegebenenfalls durch Fluor oder Chlor substituiertes Alkenyl, Alkenyloxy, Alkenylamino, Alkylidenamino, Alkenyloxycarbonyl, Alkinyl, Alkinyloxy, Alkinylamino oder Alkinyloxycarbonyl mit jeweils 2 bis 6 Kohlenstoffatomen in den Alkenyl-, Alkyliden- oder Alkinylgruppen steht,
A³ weiterhin für jeweils gegebenenfalls durch Fluor, Chlor, Cyano, Carboxy, C₁-C₄-Alkyl und/oder C₁-C₄-Alkoxy-carbonyl substituiertes Cycloalkyl, Cycloalkyloxy, Cycloalkylalkyl, Cycloalkylalkoxy, Cycloalkylidenamino, Cycloalkyloxycarbonyl oder Cycloalkylalkoxycarbonyl mit jeweils 3 bis 6 Kohlenstoffatomen in den Cycloalkylgruppen und gegebenenfalls 1 bis 4 Kohlenstoffatomen in den Alkylgruppen steht,
A³ weiterhin für jeweils gegebenenfalls durch Nitro, Cyano, Carboxy, Fluor, Chlor, Brom, C₁-C₄-Alkyl, C₁-C₄-Halogenalkyl, C₁-C₄-Alkyloxy, C₁-C₄-Halogenalkyloxy und/oder C₁-C₄-Alkoxy-carbonyl substituiertes Phenyl, Phenyloxy, Phenyl-C₁-C₄-alkyl, Phenyl-C₁-C₄-alkoxy, Phenyloxycarbonyl oder Phenyl-C₁-C₄-alkoxycarbonyl steht,
A³ weiterhin für jeweils gegebenenfalls ganz oder teilweise hydriertes Pyrrolyl, Pyrazolyl, Imidazolyl, Triazolyl, Furyl, Oxiranyl, Oxetanyl, Dioxolanyl, Thienyl, Oxazolyl, Isoxazolyl, Thiazolyl, Isothiazolyl, Oxadiazolyl, Thiadiazolyl, Pyridinyl, Pyrimidinyl, Triazinyl, Pyrazolyl-C₁-C₄-alkyl, Furyl-C₁-C₄-alkyl, Thienyl-C₁-C₄-alkyl, Oxazolyl-C₁-C₄-alkyl, Isoxazolyl-C₁-C₄-alkyl, Thiazolyl-C₁-C₄-alkyl, Pyridinyl-C₁-C₄-alkyl, Pyrimidinyl-C₁-C₄-alkyl, Pyrazolylmethoxy, Furylmethoxy, für Perhydropyranylmethoxy oder Pyridylmethoxy steht, und
- R⁷: für Wasserstoff, Fluor oder Chlor steht.

Man erhält die neuen substituierten N-Aryl-Stickstoffheterocyclen der allgemeinen Formel (I), wenn man
(a) (Thio)Semicarbazid-Derivate der allgemeinen Formel (II) 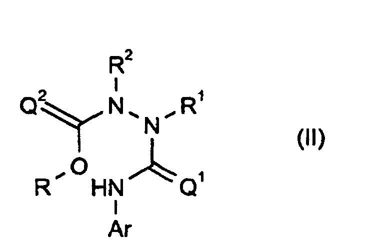 in welcher
   - Q¹, Q², R¹, R² und Ar: die oben angegebenen Bedeutungen haben und
   - R: für Alkyl steht,
   gegebenenfalls in Gegenwart eines Reaktionshilfsmittels und gegebenenfalls in Gegenwart eines Verdünnungsmittels cyclisierend kondensiert und gegebenenfalls im Anschluß daran im Rahmen der Substituentendefinition auf übliche Weise elektrophile oder nucleophile Substitutionsreaktionen durchführt,
   oder wenn man
(b) Aryliminoheterocyclen der allgemeinen Formel (III) 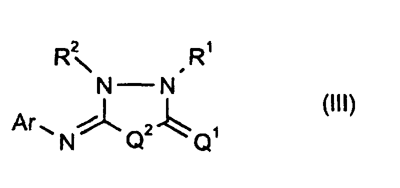 in welcher
   - Q¹, Q², R¹, R² und Ar: die oben angegebenen Bedeutungen haben,
   gegebenenfalls in Gegenwart eines Reaktionshilfsmittels und gegebenenfalls in Gegenwart eines Verdünnungsmittels thermisch ("pyrolytisch") isomerisiert.
   Die Verbindungen der Formel (I) können prinzipiell auch wie im Folgenden schematisch dargestellt synthetisiert werden:
(c) Umsetzung von Aryliso(thio)cyanaten der Formel (IV) mit Hydrazinen der Formel (V) zu Aryl(thio)semicarbaziden der Formel (VI) und deren Umsetzung mit (Thio)Phosgen: 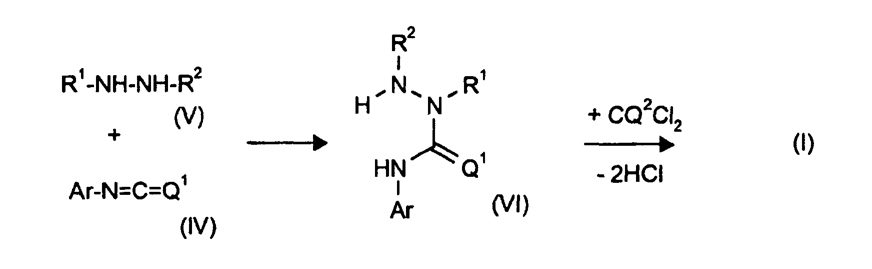
(d) Umsetzung von Aryliso(thio)cyanaten der Formel (IV) mit S-Alkyldithiocarbazaten der Formel (VII) und anschließende cyclisierende Kondensation: 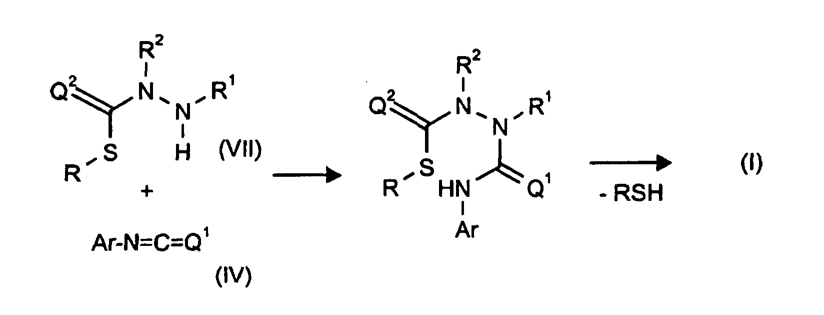
(e) Umsetzung von N,N-Bis-chlorcarbonyl- oder N,N-Bis-phenoxycarbonyl-arylaminen der Formel (VIII) - Y: Cl oder OC₆H₅ - mit Hydrazinen der Formel (V): 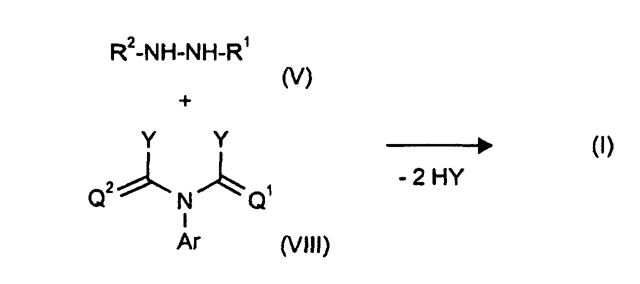
(f) Umsetzung von Arylaminen der Formel (IX) mit Hydrazindicarbonsäureestern der Formel (X):

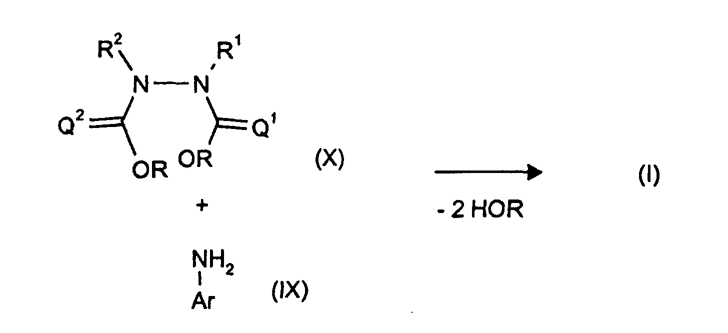

Die Verbindungen der allgemeinen Formel (I) können auch nach weiteren üblichen Methoden in andere Verbindungen der allgemeinen Formel (I) gemäß obiger Definition umgewandelt werden, beispielsweise durch übliche Umwandlungen von Carbonsäure-Gruppierungen oder deren Derivaten (z.B. R⁵: COOH → COCl, COOH → COOCH₃, COCl → CONH₂, COOCH₃ → CONH₂, CONH₂ → CN, CN → CSNH₂), durch Alkylierungsreaktionen (z.B. R¹: H → CH₃ oder CHF₂) oder durch Oxidation oder Schwefelung (z.B. Q¹: O → S oder S → O) - vgl. auch die Herstellungsbeispiele.

Die neuen substituierten N-Aryl-Stickstoffheterocyclen der allgemeinen Formel (I) zeichnen sich durch starke herbizide Wirksamkeit aus.

In den Definitionen sind die gesättigten oder ungesättigten Kohlenwasserstoffketten, wie Alkyl, Alkenyl oder Alkinyl jeweils geradkettig oder verzweigt.

Halogen steht im allgemeinen für Fluor, Chlor, Brom oder Iod, vorzugsweise für Fluor, Chlor oder Brom, insbesondere für Fluor oder Chlor.

Die Erfindung betrifft insbesondere Verbindungen der Formel (I), in welcher
- Q¹: für Sauerstoff oder Schwefel steht,
- Q²: für Sauerstoff oder Schwefel steht,
- R¹: für gegebenenfalls durch Fluor oder Chlor substituiertes Methyl, Ethyl, n- oder i-Propyl, n-, i-, s- oder t-Butyl steht,
- R²: für gegebenenfalls durch Fluor oder Chlor substituiertes Methyl, Ethyl, n- oder i-Propyl, n-, i-, s- oder t-Butyl steht, und
- Ar: für die nachstehend definierte substituierte, monocyclische Aryl-Gruppierung steht, 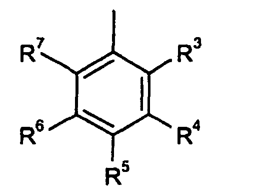 worin
R³ für Fluor oder Chlor steht,
R⁴ für Wasserstoff, Fluor oder Chlor steht,
R⁵ für Cyano, Thiocarbamoyl, Chlor, Brom, Methyl, Trifluormethyl, Methoxy, Difluormethoxy oder Trifluormethoxy steht,
R⁶ für die nachstehende Gruppierung steht,

-A¹-A²-A³

in welcher
A¹ für eine Einfachbindung oder für Sauerstoff, Schwefel, -SO-, -SO₂-, -CO- oder die Gruppierung -N-A⁴- steht, worin A⁴ für Wasserstoff, Hydroxy, Methyl, Ethyl, n- oder i-Propyl, Methoxy, Ethoxy, n- oder i-Propoxy, Methylsulfonyl oder Ethylsulfonyl steht,
A¹ weiterhin für Methylen, Ethan-1,1-diyl, Ethan-1,2-diyl, Propan-1,1-diyl, Propan-1,2-diyl, Propan-1,3-diyl, Ethen-1,2-diyl, Propen-1,2-diyl, Propen-1,3-diyl, Ethin-1,2-diyl, Propin-1,2-diyl oder Propin-1,3-diyl steht,
A² für eine Einfachbindung oder für Sauerstoff, Schwefel, -SO-, -SO₂-, -CO- oder die Gruppierung -N-A⁴- steht, worin A⁴ für Wasserstoff, Hydroxy, Methyl, Ethyl, n- oder i-Propyl, Methoxy, Ethoxy, n- oder i-Propoxy, Methylsulfonyl, Ethylsulfonyl, n- oder i-Propylsulfonyl oder Phenylsulfonyl steht,
A² weiterhin für Methylen, Ethan-1,1-diyl, Ethan-1,2-diyl, Propan-1,1-diyl, Propan-1,2-diyl, Propan-1,3-diyl, Ethen-1,2-diyl, Propen-1,2-diyl, Propen-1,3-diyl, Ethin-1,2-diyl, Propin-1,2-diyl oder Propin-1,3-diyl steht,
A³ für Wasserstoff steht, mit der Maßgabe, daß in diesem Fall A¹ und/oder A² nicht für eine Einfachbindung stehen,
A³ weiterhin für Hydroxy, Amino, Cyano, Nitro, Carboxy, Carbamoyl, Sulfo, Fluor, Chlor, Brom steht,
A³ weiterhin für jeweils gegebenenfalls durch Fluor, Chlor, Methoxy oder Ethoxy substituiertes Methyl, Ethyl, n- oder i-Propyl, n-, i-, s- oder t-Butyl, n-, i-, s- oder t-Pentyl, Methoxy, Ethoxy, n- oder i-Propoxy, n-, i-, s- oder t-Butoxy, n-, i-, s- oder t-Pentyloxy, Methylthio, Ethylthio, n- oder i-Propylthio, n-, i-, s- oder t-Butylthio, Methylsulfinyl, Ethylsulfinyl, n- oder i-Propylsulfinyl, Methylsulfonyl, Ethylsulfonyl, n- oder i-Propylsulfonyl, Methylamino, Ethylamino, n- oder i-Propylamino, n-, i-, s- oder t-Butylamino, Dimethylamino, Diethylamino, Methoxycarbonyl, Ethoxycarbonyl, n- oder i-Propoxycarbonyl, Dimethoxyphosphoryl, Diethoxyphosphoryl, Dipropoxyphosphoryl oder Diisopropoxyphosphoryl steht,
A³ weiterhin für jeweils gegebenenfalls durch Fluor oder Chlor substituiertes Propenyl, Butenyl, Propenyloxy, Butenyloxy, Propenylamino, Butenylamino, Propylidenamino, Butylidenamino, Propenyloxycarbonyl, Butenyloxycarbonyl, Propinyl, Butinyl, Propinyloxy, Butinyloxy, Propinylamino, Butinylamino, Propinyloxycarbonyl oder Butinyloxycarbonyl steht,
A³ weiterhin für jeweils gegebenenfalls durch Fluor, Chlor, Cyano, Carboxy, Methyl, Ethyl, n- oder i-Propyl, Methoxycarbonyl oder Ethoxycarbonyl substituiertes Cyclopropyl, Cyclobutyl, Cyclopentyl, Cyclohexyl, Cyclopropyloxy, Cyclobutyloxy, Cyclopentyloxy, Cyclohexyloxy, Cyclopropylmethyl, Cyclobutylmethyl, Cyclopentylmethyl, Cyclohexylmethyl, Cyclo-propylmethoxy, Cyclobutylmethoxy, Cyclopentylmethoxy, Cyclohexylmethoxy, Cyclopentylidenamino, Cyclohexylidenamino, Cyclopentyloxycarbonyl, Cyclohexyloxycarbonyl, Cyclopentylmethoxycarbonyl oder Cyclohexylmethoxycarbonyl steht,
A³ weiterhin für jeweils gegebenenfalls durch Nitro, Cyano, Carboxy, Fluor, Chlor, Brom, Methyl, Ethyl, n- oder i-Propyl, Trifluormethyl, Methoxy, Ethoxy, n- oder i-Propoxy, Difluormethoxy, Trifluormethoxy, Methoxycarbonyl und/oder Ethoxycarbonyl substituiertes Phenyl, Phenyloxy, Benzyl, Phenylethyl, Benzyloxy, Phenyloxycarbonyl, Benzyloxycarbonyl steht,
A³ weiterhin für jeweils gegebenenfalls ganz oder teilweise hydriertes Pyrrolyl, Pyrazolyl, Imidazolyl, Triazolyl, Furyl, Thienyl, Oxazolyl, Isoxazolyl, Thiazolyl, Isothiazolyl, Oxadiazolyl, Thiadiazolyl, Pyridinyl, Pyrimidinyl, Triazinyl, Pyrazolylmethyl, Furylmethyl, Thienylmethyl, Oxazolylmethyl, Isoxazolylmethyl, Thiazolylmethyl, Pyridinylmethyl, Pyrimidinylmethyl, Pyrazolylmethoxy, Furylmethoxy oder Pyridylmethoxy steht,
R⁷ für Wasserstoff, Fluor oder Chlor steht.

Die oben aufgeführten allgemeinen oder in Vorzugsbereichen angegebenen Restedefinitionen gelten sowohl für die Endprodukte der Formel (I) als auch entsprechend für die jeweils zu Herstellung benötigten Ausgangstoffe bzw. Zwischenprodukte. Diese Restedefinitionen können untereinander, also auch zwischen den angegebenen Bereichen bevorzugter Verbindungen, beliebig kombiniert werden.

Beispiele für die erfindungsgemäßen Verbindungen der Formel (I) sind in den nachstehenden Gruppen aufgeführt.

### Gruppe 1

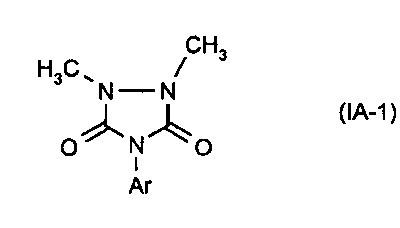

Ar hat hierbei beispielhaft die im Folgenden aufgeführten Bedeutungen:
2,4,5-Trichlor-phenyl, 2,4-Dichlor-5-fluor-phenyl, 2-Chlor-4,5-difluor-phenyl, 4-Chlor-2,5-difluor-phenyl, 5-Chlor-2,4-difluor-phenyl, 2-Fluor-5-chlor-4-cyano-phenyl, 2,4,5-Trifluor-phenyl, 2,5-Dichlor-4-cyano-phenyl, 2-Chlor-5-fluor-4-cyano-phenyl, 2-Chlor-4,5-dicyano-phenyl, 2-Chlor-4-fluor-5-cyano-phenyl, 2,5-Difluor-4-cyano-phenyl, 2-Chlor-4-cyano-5-methyl-phenyl, 2,4-Dichlor-5-methoxy-phenyl, 2,4-Dichlor-5-ethoxyphenyl, 2,4-Dichlor-5-n-propoxy-phenyl, 2,4-Dichlor-5-i-propoxy-phenyl, 4-Chlor-2-fluor-5-methoxy-phenyl, 4-Chlor-2-fluor-5-ethoxy-phenyl, 4-Chlor-2-fluor-5-n-propoxy-phenyl, 4-Chlor-2-fluor-5-i-propoxy-phenyl, 2-Fluor-4-cyano-5-methyl-phenyl, 2,4-Dichlor-5-methyl-phenyl, 2-Chlor-4-cyano-5-trifluormethyl-phenyl, 2-Fluor-4-cyano-5-trifluormethyl-phenyl, 2-Chlor-4-methyl-5-trifluormethyl-phenyl, 2-Chlor-5-fluor-4-methoxy-phenyl, 2-Fluor-4-methoxy-5-methyl-phenyl, 2,5-Difluor-4-thiocarbamoyl-phenyl, 2-Chlor-4-fluor-5-i-propoxy-phenyl, 2-Fluor-4-cyano-5-methoxy-phenyl, 2-Fluor-4-cyano-5-i-propoxyphenyl, 2-Chlor-4-cyano-5-(2-propinyloxy)-phenyl, 2-Fluor-4-cyano-5-(1-methyl-2-propinyloxy)-phenyl, 2-Fluor-4-chlor-5-(1-methyl-2-propinyloxy)-phenyl, 2-Chlor-4-thiocarbamoyl-5-i-propoxy-phenyl, 2-Fluor-4-cyano-5-(2-propenyloxy)-phenyl, 2-Fluor-4-chlor-5-(2-propenyloxy)-phenyl, 2-Chlor-4-cyano-5-methylsulfonylaminophenyl, 2-Fluor-4-cyano-5-ethylsulfonylamino-phenyl, 2-Fluor-4-thiocarbamoyl-5-methylsulfonylamino-phenyl, 2-Chlor-4-cyano-5-ethylsulfonylamino-phenyl, 2-Fluor-4-cyano-5-cyclopropylsulfonylamino-phenyl, 2-Fluor-4-cyano-5-i-propylsulfonylamino-phenyl, 2-Chlor-4-thiocarbamoyl-5-ethylsulfonylamino-phenyl, 2-Chlor-4-cyano-5-cyanamino-phenyl, 2-Fluor-4-cyano-5-(2,2-difluorethylsulfonylamino)-phenyl, 2-Fluor-4-cyano-5-phenylsulfonylamino-phenyl, 2-Fluor-4-cyano-5-t-butylsulfonylamino-phenyl, 2-Chlor-4-cyano-5-methoxycarbonyl-phenyl, 2-Fluor-4-cyano-5-ethoxycarbonyl-phenyl, 2-Fluor-4-chlor-5-ethoxycarbonyl-phenyl, 2-Fluor-4-thiocarbamoyl-5-methoxycarbonyl-phenyl, 2-Chlor-4-cyano-5-(N-cyclopropylethylsulfonylamino)-phenyl, 2-Fluor-4-cyano-5-(1-methyl-2-propinylthio)-phenyl, 2-Fluor-4-cyano-5-methylamino-phenyl, 2-Chlor-4-thiocarbamoyl-5-methoxycarbonyl-methyl-phenyl, 2-Chlor-4-cyano-5-(N-methyl-ethylsulfonylamino)-phenyl, 2-Fluor-4-cyano-5-i-propoxycarbonyl-phenyl, 2-Fluor-4-chlor-5-i-propoxycarbonyl-phenyl, 2-Fluor-4-cyano-5-(bis-ethylsulfonyl-amino)-phenyl, 2-Fluor-4-cyano-5-(N-methylsulfonyl-ethylsulfonylamino)-phenyl, 2-Fluor-4-cyano-5-(1-methoxycarbonyl-ethoxy)-phenyl, 2-Fluor-4-cyano-5-(1-ethoxycarbonyl-ethoxy)-phenyl, 2-Fluor-4-chlor-5-(1-methoxycarbonyl-ethoxy)-phenyl, 2-Fluor-4-chlor-5-(1-ethoxycarbonyl-ethoxy)-phenyl, 2-Fluor-4-cyano-5-cyclopropyloxy-phenyl, 2-Chlor-4-cyano-5-dimethylamino-phenyl, 2-Fluor-4-cyano-5-tetrahydrofurylmethoxyphenyl, 4-Chlor-2-fluor-5-tetrahydrofurylmethoxy-phenyl, 2-Fluor-4-cyano-5-aminophenyl, 2-Fluor-4-cyano-5-methylaminocarbonyl-phenyl, 2-Fluor-4-cyano-5-methylsulfonyloxyphenyl, 2-Chlor-4-cyano-5-difluormethoxy-phenyl, 2-Fluor-4-chlor-5-methoxycarbonylmethoxy-phenyl, 2-Fluor-4-chlor-5-ethoxycarbonylmethoxyphenyl, 2-Fluor-4-cyano-5-methoxycarbonylmethoxy-phenyl, 2-Fluor-4-cyano-5-ethoxycarbonylmethoxy-phenyl, 2-Fluor-4-cyano-5-dimethylaminocarbonyl-phenyl, 2-Fluor-4-cyano-5-cyanomethoxyphenyl, 2-Fluor-4-cyano-5-(2-chlor-2-propenyloxy)-phenyl, 2-Fluor-4-cyano-5-hydroxy-phenyl, 2-Fluor-4-cyano-5-nitro-phenyl, 2-Fluor-4-cyano-5-diethoxyphosphorylamino-phenyl, 2-Fluor-4-cyano-5-chlor-sulfonyl-phenyl, 2-Fluor-4-cyano-5-formylamino-phenyl, 2-Chlor-4-cyano-5-ethoxycarbonyloxy-phenyl, 2-Fluor-4-cyano-5-diethoxyphosphorylmethoxy-phenyl, 4-Chlor-2-fluor-5-diethoxyphosphorylmethoxy-phenyl, 2-Fluor-4-cyano-5-(1-diethoxyphosphoryl-ethoxy)-phenyl-, 4-Chlor-2-fluor-5-(1-diethoxyphosphorylethoxy)-phenyl-, 2-Chlor-4-cyano-5-hydroxy-phenyl, 2-Fluor-4-cyano-5-(N,N-diacetyl-amino)-phenyl, 2-Fluor-4-cyano-5-acetylamino-phenyl, 2-Chlor-4-cyano-5-thiocyanato-phenyl, 2-Fluor-4-cyano-5-diethylaminooxy-phenyl, 2-Fluor-4-cyano-5-tetrahydrofuryloxy-phenyl, 2-Fluor-4-cyano-5-ureido-phenyl, 2-Fluor-4-cyano-5-dimethoxymethylenaminophenyl, 2-Chlor-4-cyano-5-ethoxymethylenamino-phenyl, 2-Fluor-4-cyano-5-(2-chlor-ethoxycarbonyloxy)-phenyl, 2-Chlor-4-cyano-5-dimethylaminomethylenamino-phenyl, 2-Chlor-4-cyano-5-(perhydropyran-4-yloxy)-phenyl, 2-Fluor-4-cyano-5-(2-methoxycarbonyl-ethyl)-phenyl, 4-Chlor-2-fluor-5-(2-methoxycarbonyl-ethyl)-phenyl, 2-Chlor-4-cyano-5-(2-carboxy-2-chlor-ethyl)-phenyl, 2-Fluor-4-cyano-5-(2-chlor-2-methoxycarbonyl-ethyl)-phenyl, 2-Fluor-4-chlor-5-(2-chlor-2-methoxycarbonyl-ethyl)-phenyl, 2-Fluor-4-cyano-5-(2-chlor-2-ethoxycarbonyl-ethyl)-phenyl, 2-Fluor-4-chlor-5-(2-chlor-2-ethoxycarbonyl-ethyl)-phenyl, 2-Fluor-4-cyano-5-(2-brom-2-methoxycarbonyl-ethyl)-phenyl, 2-Fluor-4-chlor-5-(2-brom-2-methoxycarbonyl-ethyl)-phenyl, 2-Fluor-4-cyano-5-(2-brom-2-ethoxycarbonyl-ethyl)-phenyl, 2-Fluor-4-chlor-5-(2-brom-2-ethoxycarbonyl-ethyl)-phenyl, 2-Fluor-4-cyano-5-(2,3-dibrom-2-methoxycarbonyl-ethyl)-phenyl, 2-Fluor-4-chlor-5-(2,3-dibrom-2-methoxycarbonyl-ethyl)-phenyl, 2-Fluor-4-cyano-5-(2,3-dibrom-2-ethoxycarbonyl-ethyl)-phenyl, 2-Fluor-4-chlor-5-(2,3-dibrom-2-ethoxycarbonyl-ethyl)-phenyl, 2-Fluor-4-cyano-5-(2-chlor-2-s-butoxycarbonyl)-phenyl, 2-Fluor-4-cyano-5-(2-chlor-2-carbamoyl-ethyl)-phenyl, 2-Fluor-4-cyano-5-(2-chlor-2-methoxycarbonyl-1-methyl-ethyl)-phenyl, 2-Fluor-4-cyano-5-(1,2-dibrom-2-methoxycarbonyl-ethyl)-phenyl, 2-Chlor-4-cyano-5-(2-chlor-2-i-propoxy-carbonyl-ethyl)-phenyl, 2,4-Dichlor-5-(2-methoxycarbonyl-ethyl)-phenyl, 2-Fluor-4-cyano-5-(2-carboxy-2-chlor-ethyl)-phenyl, 2-Fluor-4-cyano-5-(2-chlor-2-ethylaminocarbonyl-ethyl)-phenyl, 2-Fluor-4-cyano-5-(2-allylaminocarbonyl-2-chlor-ethyl)-phenyl, 2-Fluor-4-cyano-5-(2-methoxycarbonyl-ethenyl)-phenyl, 2-Fluor-4-chlor-5-(2-methoxycarbonyl-ethenyl)-phenyl, 2-Fluor-4-cyano-5-(2-ethoxycarbonyl-ethenyl)-phenyl, 2-Fluor-4-chlor-5-(2-ethoxycarbonyl-ethenyl)-phenyl, 2-Fluor-4-cyano-5-(2-chlor-2-methylaminocarbonyl-ethyl)-phenyl, 2-Fluor-4-cyano-5-(2-chlor-2-ethylaminocarbonyl-ethyl)-phenyl, 2-Fluor-4-cyano-5-(2-chlor-2-cyclopropylamino-carbonyl-ethyl)-phenyl, 2-Fluor-4-chlor-5-(2-chlor-2-methylaminocarbonyl-ethyl)-phenyl, 2-Fluor-4-chlor-5-(2-chlor-2-ethylaminocarbonyl-ethyl)-phenyl, 2-Fluor-4-chlor-5-(2-chlor-2-cyclopropylaminocarbonyl-ethyl)-phenyl, 2-Fluor-4-cyano-5-(2-chlor-2-dimethylaminocarbonyl-ethyl)-phenyl, 2-Fluor-4-cyano-5-(2-chlor-2-ethylsulfonylaminocarbonyl-ethyl)-phenyl, 2-Fluor-4-chlor-5-(2-chlor-2-ethylsulfonylaminocarbonyl-ethyl)-phenyl, 2-Fluor-4-chlor-5-(2-carboxy-ethenyl)-phenyl, 2-Fluor-4-thiocarbamoyl-5-(2-ethylaminocarbonyl-ethenyl)-phenyl, 2,6-Difluor-4-cyano-5-i-propoxy-phenyl, 2-Chlor-4-cyano-6-fluor-3-i-propoxy-phenyl, 2-Chlor-6-fluor-3-i-propoxy-4-trifluormethyl-phenyl, 2,6-Dichlor-4-cyano-3-fluor-phenyl, 2-Fluor-4-cyano-5-(1-ethoxycarbonyl-ethyl)-phenyl, 2-Chlor-4-cyano-5-(1-ethoxycarbonylethyl)-phenyl, 2-Chlor-4-cyano-5-carboxy-phenyl, 2-Fluor-4-chlor-5-(1-ethoxycarbonyl-ethyl)-phenyl, 2-Fluor-4-chlor-5-(1-i-propoxycarbonyl-ethyl)-phenyl, 2-Fluor-4-cyano-5-i-butoxyphenyl, 2-Chlor-4-cyano-5-i-butoxy-phenyl, 2-Chlor-4-cyano-5-(2-methoxy-ethoxy)-phenyl, 2-Fluor-4-chlor-5-(2-methoxy-ethoxy)-phenyl, 2-Fluor-4-chlor-5-i-butoxyphenyl, 4-Hydroxy-4-ethoxycarbonylphenyl, 2-Fluor-4-cyano-5-i-propoxycarbonylphenyl, 2-Fluor-4-hydroxy-5-i-propoxycarbonyl-phenyl, 2-Fluor-4-cyano-5-(2-oxetanyloxy)-phenyl, 2-Fluor-4-cyano-5-(2-oxetanyloxy)-phenyl, 2-Fluor-4-cyano-5-(2-chlor-2-propenyloxy)-phenyl, 4-Chlor-2-fluor-5-(2-chlor-2-propenyloxy)-phenyl, 2-Fluor-4-chlor-5-methoxycarbonylmethylthio-phenyl, 2-Fluor-4-chlor-5-ethoxycarbonylmethylthio-phenyl, 2-Fluor-4-cyano-5-methoxycarbonylmethylthio-phenyl, 2-Fluor-4-cyano-5-ethoxycarbonylmethylthio-phenyl, 2-Fluor-4-chlor-5-(1-methoxycarbonyl-ethylthio)-phenyl, 2-Fluor-4-chlor-5-(1-ethoxycarbonyl-ethylthio)-phenyl, 2-Fluor-4-cyano-5-(1-methoxycarbonyl-ethylthio)-phenyl, 2-Fluor-4-cyano-5-(1-ethoxycarbonyl-ethylthio)-phenyl,

### Gruppe 2

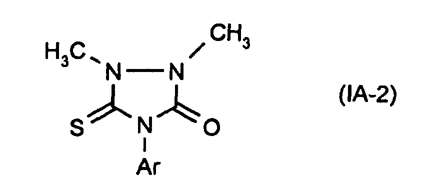

Ar hat hierbei beispielhaft die oben in Gruppe 1 aufgeführten Bedeutungen.

### Gruppe 3

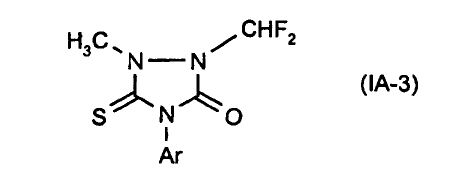

Ar hat hierbei beispielhaft die oben in Gruppe 1 aufgeführten Bedeutungen.

### Gruppe 4

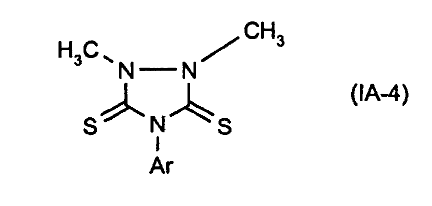

Ar hat hierbei beispielhaft die oben in Gruppe 1 aufgeführten Bedeutungen.

### Gruppe 5

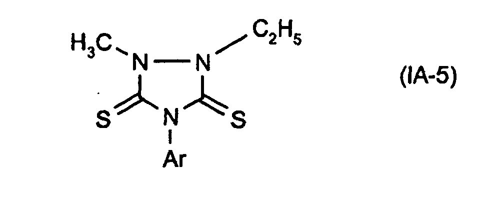

Ar hat hierbei beispielhaft die oben in Gruppe 1 aufgeführten Bedeutungen.

### Gruppe 6

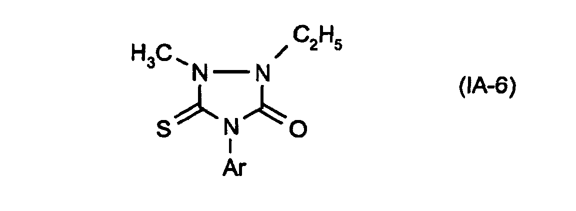

Ar hat hierbei beispielhaft die oben in Gruppe 1 aufgeführten Bedeutungen.

### Gruppe 7

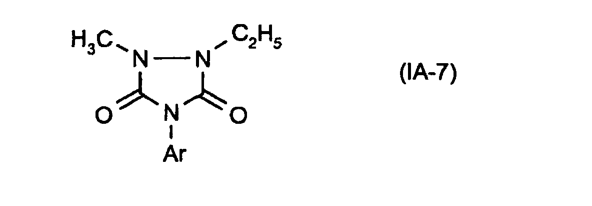

Ar hat hierbei beispielhaft die oben in Gruppe 1 aufgeführten Bedeutungen.

### Gruppe 8

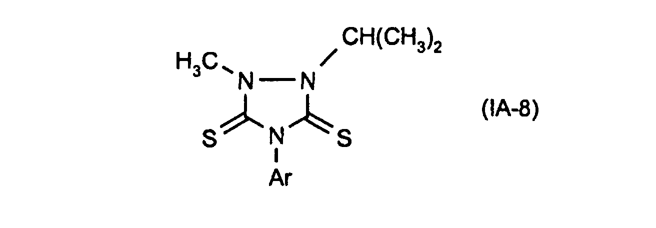

Ar hat hierbei beispielhaft die oben in Gruppe 1 aufgeführten Bedeutungen.

### Gruppe 9

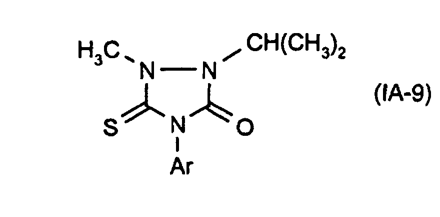

Ar hat hierbei beispielhaft die oben in Gruppe 1 aufgeführten Bedeutungen.

### Gruppe 10

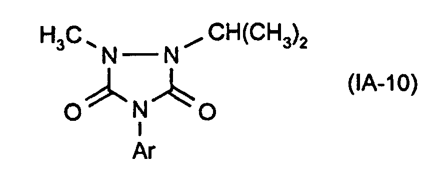

Ar hat hierbei beispielhaft die oben in Gruppe 1 aufgeführten Bedeutungen.

### Gruppe 11

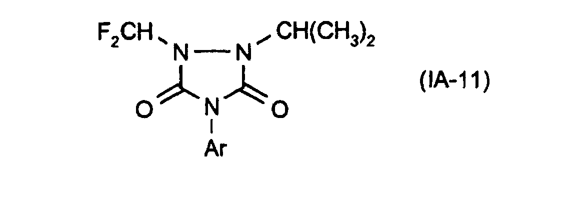

Ar hat hierbei beispielhaft die oben in Gruppe 1 aufgeführten Bedeutungen.

### Gruppe 12

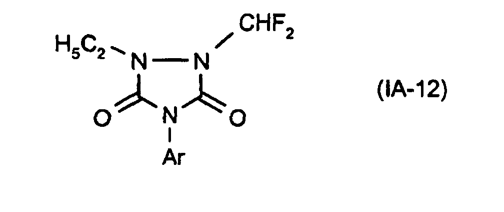

Ar hat hierbei beispielhaft die oben in Gruppe 1 aufgeführten Bedeutungen.

### Gruppe 13

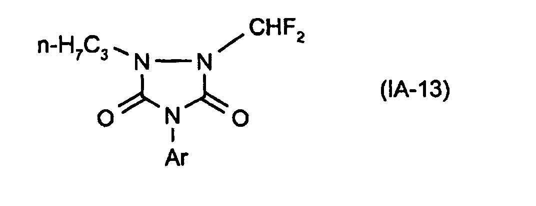

Ar hat hierbei beispielhaft die oben in Gruppe 1 aufgeführten Bedeutungen.

### Gruppe 14

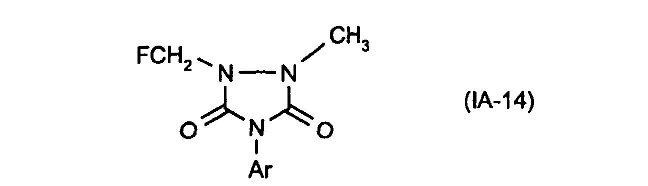

Ar hat hierbei beispielhaft die oben in Gruppe 1 aufgeführten Bedeutungen.

### Gruppe 15

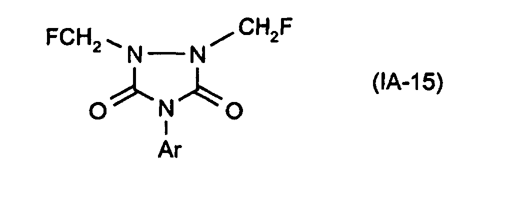

Ar hat hierbei beispielhaft die oben in Gruppe 1 aufgeführten Bedeutungen.

### Gruppe 16

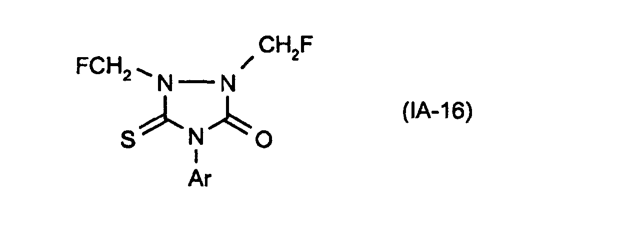

Ar hat hierbei beispielhaft die oben in Gruppe 1 aufgeführten Bedeutungen.

### Gruppe 17

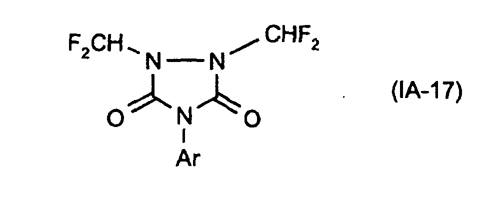

Ar hat hierbei beispielhaft die oben in Gruppe 1 aufgeführten Bedeutungen.

### Gruppe 18

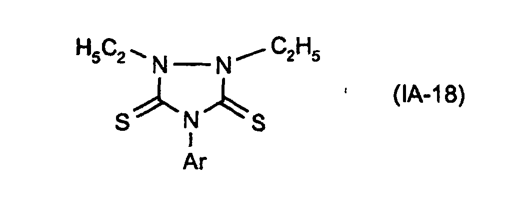

Ar hat hierbei beispielhaft die oben in Gruppe 1 aufgeführten Bedeutungen.

### Gruppe 19

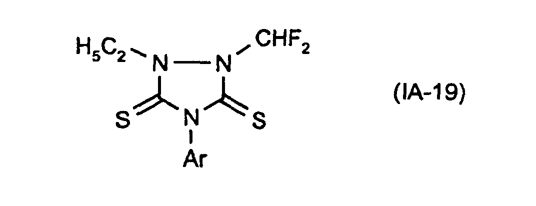

Ar hat hierbei beispielhaft die oben in Gruppe 1 aufgeführten Bedeutungen.

### Gruppe 20

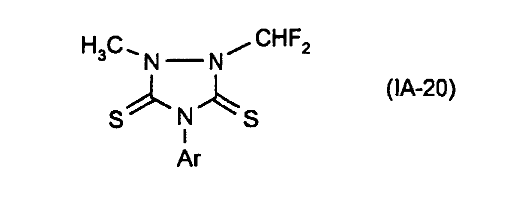

Ar hat hierbei beispielhaft die oben in Gruppe 1 aufgeführten Bedeutungen.

### Gruppe 21

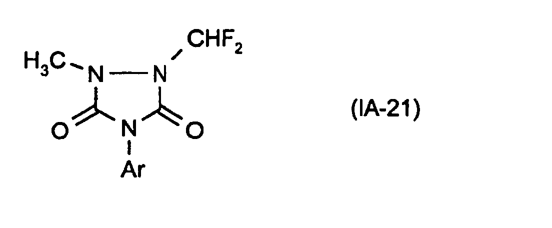

Ar hat hierbei beispielhaft die oben in Gruppe 1 aufgeführten Bedeutungen.

### Gruppe 22

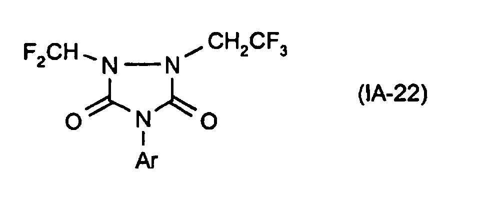

Ar hat hierbei beispielhaft die oben in Gruppe 1 aufgeführten Bedeutungen.

### Gruppe 23

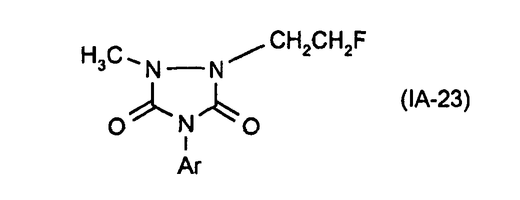

Ar hat hierbei beispielhaft die oben in Gruppe 1 aufgeführten Bedeutungen.

### Gruppe 24

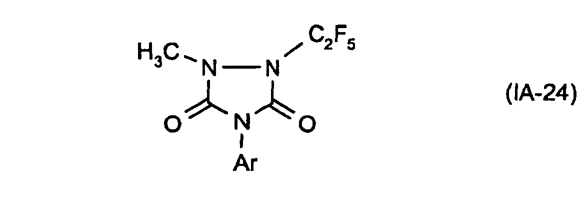

Ar hat hierbei beispielhaft die oben in Gruppe 1 aufgeführten Bedeutungen.

### Gruppe 25

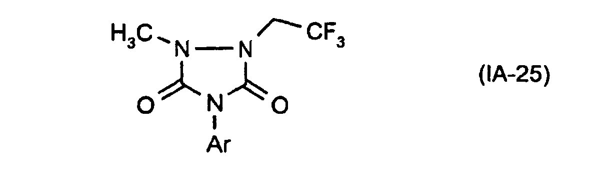

Ar hat hierbei beispielhaft die oben in Gruppe 1 aufgeführten Bedeutungen.

### Gruppe 26

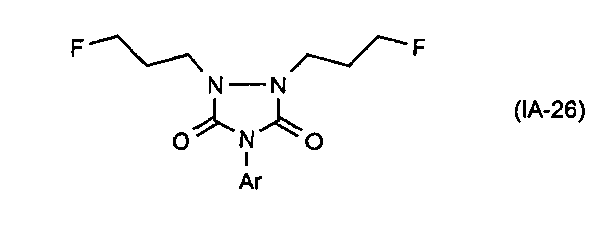

Ar hat hierbei beispielhaft die oben in Gruppe 1 aufgeführten Bedeutungen.

### Gruppe 27

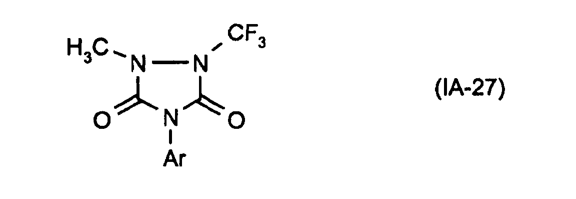

Ar hat hierbei beispielhaft die oben in Gruppe 1 aufgeführten Bedeutungen.

### Gruppe 28

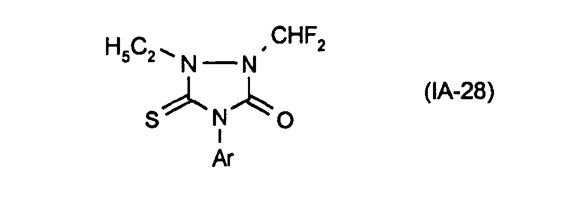

Ar hat hierbei beispielhaft die oben in Gruppe 1 aufgeführten Bedeutungen.

### Gruppe 29

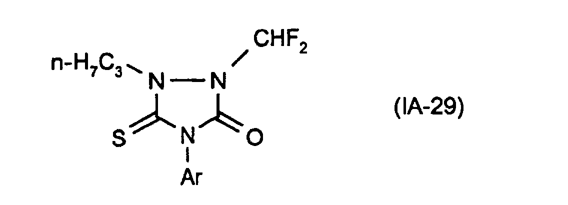

Ar hat hierbei beispielhaft die oben in Gruppe 1 aufgeführten Bedeutungen.

### Gruppe 30

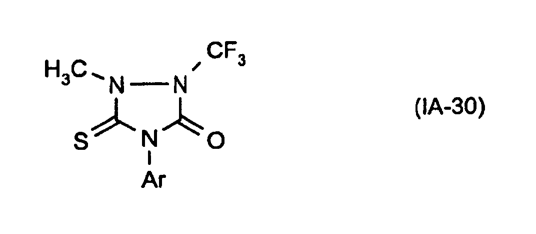

Ar hat hierbei beispielhaft die oben in Gruppe 1 aufgeführten Bedeutungen.

### Gruppe 31

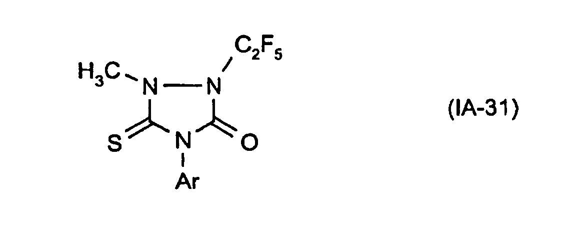

Ar hat hierbei beispielhaft die oben in Gruppe 1 aufgeführten Bedeutungen.

### Gruppe 32

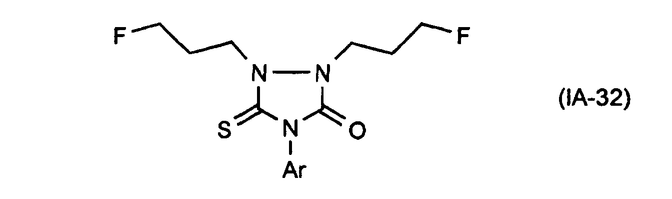

Ar hat hierbei beispielhaft die oben in Gruppe 1 aufgeführten Bedeutungen.

### Gruppe 33

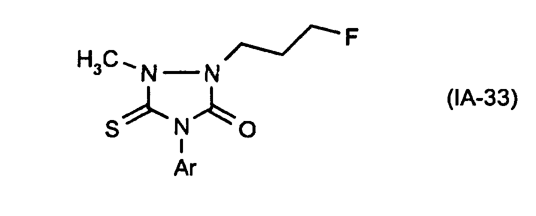

Ar hat hierbei beispielhaft die oben in Gruppe 1 aufgeführten Bedeutungen.

### Gruppe 34

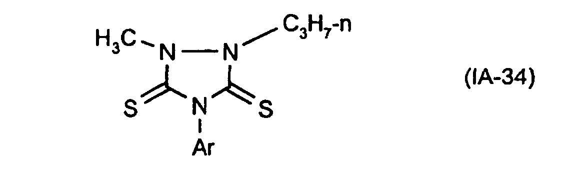

Ar hat hierbei beispielhaft die oben in Gruppe 1 aufgeführten Bedeutungen.

### Gruppe 35

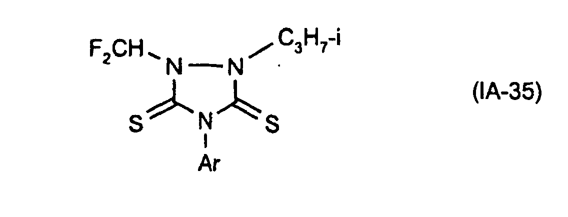

Ar hat hierbei beispielhaft die oben in Gruppe 1 aufgeführten Bedeutungen.

### Gruppe 36

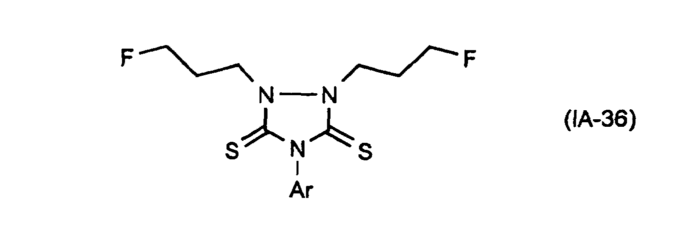

Ar hat hierbei beispielhaft die oben in Gruppe 1 aufgeführten Bedeutungen.

### Gruppe 37

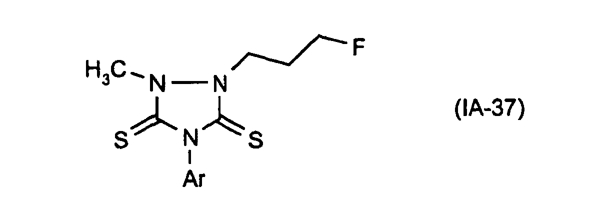

Ar hat hierbei beispielhaft die oben in Gruppe 1 aufgeführten Bedeutungen.

Verwendet man beispielsweise 4-(4-Cyano-2,5-difluor-phenyl)-1-methoxycarbonylthiosemicarbazid als Ausgangsstoff, so kann der Reaktionsablauf beim erfindungsgemäßen Verfahren (a) durch das folgende Formelschema wiedergegeben werden:

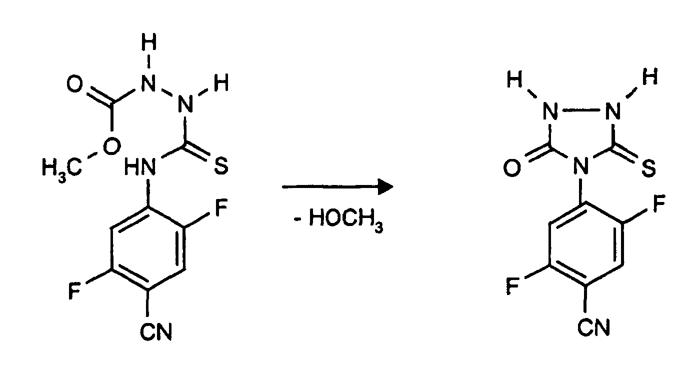

Verwendet man beispielsweise 2-(4-Chlor-2-fluor-5-methoxy-phenylimino)-3,4-dimethyl-3,4-dihydro-5-oxo-1,3,4-thiadiazol als Ausgangsstoff, so kann der Reaktionsablauf beim erfindungsgemäßen Verfahren (b) durch das folgende Formelschema skizziert werden:

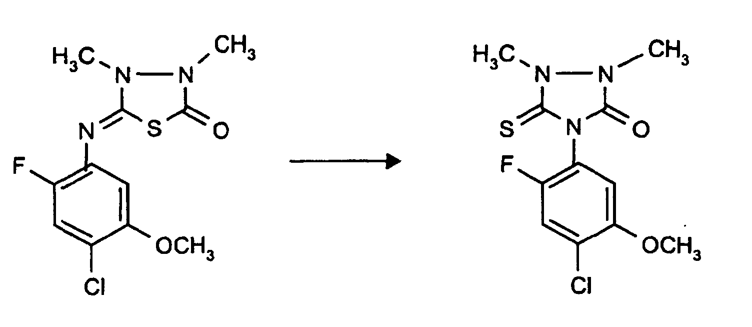

Die beim erfindungsgemäßen Verfahren (a) zur Herstellung der Verbindungen der allgemeinen Formel (I) als Ausgangsstoffe zu verwendenden (Thio)Semicarbazid-Derivate sind durch die Formel (II) allgemein definiert. In der Formel (II) haben Q¹, Q², R¹, R² und Ar vorzugsweise bzw. insbesondere diejenigen Bedeutungen, die bereits oben im Zusammenhang mit der Beschreibung der Verbindungen der Formel (I) als bevorzugt bzw. als insbesondere bevorzugt für Q¹, Q², R¹, R² und Ar angegeben wurden; R steht vorzugsweise für Alkyl mit 1 bis 4 Kohlenstoffatomen, insbesondere für Methyl oder Ethyl.

Die Ausgangsstoffe der Formel (II) sind bekannt und/oder können nach bekannten Verfahren hergestellt werden (vgl. Synthesis 1982, 159-160; DE 1200824, DE 2952685 und DE 3026739).

Man erhält die (Thio)Semicarbazid-Derivate der Formel (II), wenn man
(α) Aryliso(thio)cyanate der allgemeinen Formel (IV)

   Ar-N=C=Q¹ (IV)

   in welcher
   - Ar und Q¹: die oben angegebenen Bedeutungen haben,
   mit Carbazaten der allgemeinen Formel (XI)

   RO-CQ²-N(R²)-NH-R¹ (XI)

   in welcher
   - Q², R¹ und R²: die oben angegebenen Bedeutungen haben und
   - R: für Alkyl, vorzugsweise für C₁-C₆-Alkyl, insbesondere bevorzugt für Methyl oder Ethyl steht,
   gegebenenfalls in Gegenwart eines Verdünnungsmittels, wie z.B. Toluol, bei Temperaturen zwischen 0°C und 150°C umsetzt (vgl. die Herstellungsbeispiele),
   oder wenn man
(β) Arylamine der allgemeinen Formel (XII)

   Ar-NH₂ (XII)
in welcher
- Ar: die oben angegebene Bedeutung hat,
mit (Thio)Carbonyl-diimidazol der Formel (XIII)

Im-CQ¹-Im (XIII)

in welcher
- Q¹: die oben angegebene Bedeutung hat und
- Im: für Imidazolyl steht,
und mit Carbazaten der allgemeinen Formel (XI)

RO-CQ²-N(R²)-NH-R¹ (XI)

in welcher
- Q², R¹ und R²: die oben angegebenen Bedeutungen haben und
- R: für Alkyl, vorzugsweise für C₁-C₆-Alkyl, insbesondere bevorzugt für Methyl oder Ethyl steht,
gegebenenfalls in Gegenwart eines Reaktionshilfsmittels, wie z.B. Kaliumhydroxid, und gegebenenfalls in Gegenwart eines Verdünnungsmittels, wie z.B. Methanol, Ethanol und/oder Wasser, bei Temperaturen zwischen 0°C und 100°C umsetzt.

Die als Vorprodukte benötigten Aryliso(thio)cyanate der Formel (IV) sind bekannt und/oder können nach bekannten Verfahren hergestellt werden (vgl. DE 4327743, DE 4335438 und DE 4343451).

Die weiter als Vorprodukte benötigten Carbazate der Formel (VIII) sind bekannte organische Chemikalien.

Das erfindungsgemäße Verfahren (a) wird vorzugsweise in Gegenwart eines geeigneten Reaktionshilfsmittels durchgeführt. Als solche kommen alle üblichen anorganischen oder organischen Basen infrage. Hierzu gehören beispielsweise Alkalimetall- oder Erdalkalimetall-hydride, -hydroxide, -amide, -alkoholate, -acetate, -carbonate oder -hydrogencarbonate, wie beispielsweise Lithium-, Natrium-, Kalium- oder Calcium-hydrid, Lithium-, Natrium- oder Kalium-amid, Natrium- oder Kaliummethylat, Natrium- oder Kalium-ethylat, Natrium- oder Kalium-propylat, Aluminiumisopropylat, Natrium- oder Kalium-tert-butylat, Natrium- oder Kaliumhydroxid, Ammoniumhydroxid, Natrium-, Kalium- oder Calcium-acetat, Ammoniumacetat, Natrium-, Kalium-, Rubidium-, Cäsium-, Magnesium- oder Calcium-carbonat, Ammoniumcarbonat, Natrium- oder Kalium-hydrogencarbonat, sowie basische organische Stickstoffverbindungen, wie Trimethylamin, Triethylamin, Tripropylamin, Tributylamin, Ethyl-diisopropylamin, N,N-Dimethylcyclohexylamin, Dicyclohexylamin, Ethyl-dicyclohexylamin, N,N-Dimethylanilin, N,N-Dimethyl-benzylamin, Pyridin, 2-Methyl-, 3-Methyl- und 4-Methyl-pyridin, 2,4-Dimethyl-, 2,6-Dimethyl-, 3,4-Dimethyl- und 3,5-Dimethyl-pyridin, 5-Ethyl-2-methylpyridin, N-Methylpiperidin, N,N-Dimethylaminopyridin, Diazabicyclooctan (DABCO), Diazabicyclononen (DBN) oder Diazabicycloundecen (DBU).

Als Verdünnungsmittel zur Durchführung des erfindungsgemäßen Verfahrens (a) kommen die üblichen organischen Lösungsmittel in Betracht. Hierzu gehören insbesondere aliphatische, alicyclische oder aromatische, gegebenenfalls halogenierte Kohlenwasserstoffe, wie beispielsweise Pentan, Hexan, Heptan, Petrolether, Ligroin, Benzin, Benzol, Toluol, Xylol, Chlorbenzol, Dichlorbenzol, Cyclohexan, Methylcyclohexan, Dichlormethan, Chloroform, Tetrachlormethan; Ether, wie Diethylether, Diisopropylether, t-Butyl-methylether, t-Pentyl-methylether, Dioxan, Tetrahydrofuran, Ethylenglykol-dimethyl- oder -diethylether, Diethylenglykol-dimethylether oder -diethylether; Ketone, wie Aceton, Butanon oder Methyl-isobutyl-keton; Nitrile, wie Acetonitril, Propionitril, Butyronitril oder Benzonitril; Amide, wie N,N-Dimethylformamid, N,N-Dimethylacetamid, N-Methylformanilid, N-Methylpyrrolidon oder Hexamethylphosphorsäuretriamid; Ester wie Essigsäure-methylester, -ethylester, -n- oder -i-propylester, -n-, -i- oder -s-butylester, Sulfoxide, wie Dimethylsulfoxid; Alkohole, wie Methanol, Ethanol, n- oder i-Propanol, n-, i-, s-oder t-Butanol, Ethylenglykol-monomethylether oder -monoethylether, Diethylenglykolmonomethylether, Diethylenglykolmonoethylether; deren Gemische mit Wasser oder reines Wasser.

Die Reaktionstemperaturen können bei der Durchführung des erfindungsgemäßen Verfahrens (a) in einem größeren Bereich variiert werden. Im allgemeinen arbeitet man bei Temperaturen zwischen 0°C und +150°C, vorzugsweise bei Temperaturen zwischen 10°C und 120°C.

Das erfindungsgemäße Verfahren (a) wird im allgemeinen unter Normaldruck durchgeführt. Es ist jedoch auch möglich, unter erhöhtem oder vermindertem Druck - im allgemeinen zwischen 0,1 bar und 10 bar - zu arbeiten.

Zur Durchführung des erfindungsgemäßen Verfahrens (a) werden im allgemeinen die Ausgangsstoffe der Formel (II) in einem geeigneten Verdünnungsmittel vorgelegt und - gegebenenfalls nach Zugabe eines Reaktionshilfsmittels - bei der erforderlichen Temperatur bis zum Ende der Umsetzung gerührt. Die Aufarbeitung kann auf übliche Weise erfolgen (vgl. die Herstellungsbeispiele).

Die beim erfindungsgemäßen Verfahren (b) zur Herstellung der Verbindungen der allgemeinen Formel (I) als Ausgangsstoffe zu verwendenden Aryliminoheterocyclen sind durch die Formel (III) allgemein definiert. In der Formel (III) haben Q¹, Q², R¹, R² und Ar vorzugsweise bzw. insbesondere diejenigen Bedeutungen, die bereits oben im Zusammenhang mit der Beschreibung der Verbindungen der Formel (I) als bevorzugt bzw. als insbesondere bevorzugt für Q¹, Q², R¹, R² und Ar angegeben wurden.

Die Ausgangsstoffe der Formel (III) sind bisher noch nicht aus der Literatur bekannt; sie sind jedoch Gegenstand einer vorgängigen, nicht vorveröffentlichten Anmeldung (vgl. DE 4424787).

Man erhält die Aryliminoheterocyclen der Formel (III), wenn man Aryl(thio)semicarbazide der allgemeinen Formel (VI) mit reaktiven Kohlensäurederivaten, wie z.B. Phosgen oder Thiophosgen, gegebenenfalls in Gegenwart von Verdünnungsmitteln, wie z.B. Toluol und/oder Dichlormethan, bei Temperaturen zwischen 0°C und 100°C umsetzt.

Das erfindungsgemäße Verfahren (b) wird gegebenenfalls in Gegenwart eines Reaktionshilfsmittels durchgeführt. Es kommen hierbei die gleichen Reaktionshilfsmittel wie beim erfindungsgemäßen Verfahren (a), außerdem jedoch auch noch Alkalimetallsulfide, wie z.B. Natrium- oder Kaliumsulfid, in Betracht.

Das erfindungsgemäße Verfahren (b) wird vorzugsweise unter Verwendung eines Verdünnungsmittels durchgeführt. Es kommen hierbei die gleichen Verdünnungsmittel wie beim erfindungsgemäßen Verfahren (a) in Betracht.

Die Reaktionstemperaturen können bei der Durchführung des erfindungsgemäßen Verfahrens (b) in einem größeren Bereich variiert werden. Im allgemeinen arbeitet man bei Temperaturen zwischen 0°C und +250°C, vorzugsweise bei Temperaturen zwischen 20°C und 150°C.

Das erfindungsgemäße Verfahren (b) wird im allgemeinen unter Normaldruck durchgeführt. Es ist jedoch auch möglich, unter erhöhtem oder vermindertem Druck - im allgemeinen zwischen 0,1 bar und 10 bar - zu arbeiten.

Zur Durchführung des erfindungsgemäßen Verfahrens (b) werden im allgemeinen die Ausgangsstoffe der Formel (III) in einem geeigneten Verdünnungsmittel vorgelegt und bei der erforderlichen Temperatur bis zum Ende der Umsetzung gerührt. Die Aufarbeitung kann auf übliche Weise erfolgen.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewandten Menge ab.

Die erfindungsgemäßen Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea, Trifolium, Ranunculus, Taraxacum.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen, auf Zier- und Sportrasen und Weideflächen und zur selektiven Unkrautbekämpfung in einjährigen Kulturen eingesetzt werden.

Die erfindungsgemäßen Verbindungen der Formel (I) eignen sich insbesondere zur selektiven Bekämpfung von monokotylen und dikotylen Unkräutern in monokotylen Kulturen sowohl im Vorauflauf- als auch im Nachauflauf-Verfahren.

Die Wirkstoffe können in die üblichen Formulierungen übergeführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.
Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:
z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide infrage, beispielsweise Anilide, wie z.B. Diflufenican und Propanil; Arylcarbonsäuren, wie z.B. Dichlorpicolin-säure, Dicamba und Picloram; Aryloxyalkansäuren, wie z.B. 2,4-D, 2,4-DB, 2,4-DP, Fluroxypyr, MCPA, MCPP und Triclopyr; Aryloxy-phenoxy-alkansäureester, wie z.B. Diclofop-methyl, Fenoxaprop-ethyl, Fluazifop-butyl, Haloxyfop-methyl und Quizalofop-ethyl; Azinone, wie z.B. Chloridazon und Norflurazon; Carbamate, wie z.B. Chlorpropham, Desmedipham, Phenmedipham und Propham; Chloracetanilide, wie z.B. Alachlor, Acetochlor, Butachlor, Metazachlor, Metolachlor, Pretilachlor und Propachlor; Dinitroaniline, wie z.B. Oryzalin, Pendimethalin und Trifluralin; Diphenylether, wie z.B. Acifluorfen, Bifenox, Fluoroglycofen, Fomesafen, Halosafen, Lactofen und Oxyfluorfen; Harnstoffe, wie z.B. Chlortoluron, Diuron, Fluometuron, Isoproturon, Linuron und Methabenzthiazuron; Hydroxylamine, wie z.B. Alloxydim, Clethodim, Cycloxydim, Sethoxydim und Tralkoxydim; Imidazolinone, wie z.B. Imazethapyr, Imazamethabenz, Imazapyr und Imazaquin; Nitrile, wie z.B. Bromoxynil, Dichlobenil und Ioxynil; Oxyacetamide, wie z.B. Mefenacet; Sulfonylharnstoffe, wie z.B. Amidosulfuron, Bensulfuron-methyl, Chlorimuron-ethyl, Chlorsulfuron, Cinosulfuron, Metsulfuron-methyl, Nicosulfuron, Primisulfuron, Pyrazosulfuron-ethyl, Thifensulfuron-methyl, Triasulfuron und Tribenuron-methyl; Thiolcarbamate, wie z.B. Butylate, Cycloate, Diallate, EPTC, Esprocarb, Molinate, Prosulfocarb, Thiobencarb und Triallate; Triazine, wie z.B. Atrazin, Cyanazin, Simazin, Simetryne, Terbutryne und Terbutylazin; Triazinone, wie z.B. Hexazinon, Metamitron und Metribuzin; Sonstige, wie z.B. Aminotriazol, Benfuresate, Bentazone, Cinmethylin, Clomazone, Clopyralid, Difenzoquat, Dithiopyr, Ethofumesate, Fluorochloridone, Glufosinate, Glyphosate, Isoxaben, Pyridate, Quinchlorac, Quinmerac, Sulphosate und Tridiphane.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden. Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 1 g und 10 kg Wirkstoff pro Hektar Bodenfläche, vorzugsweise zwischen 5 g und 5 kg pro ha.

Die Herstellung und die Verwendung der erfindungsgemäßen Wirkstoffe geht aus den nachfolgenden Beispielen hervor.

### Herstellungsbeispiele:

### Beispiel 1

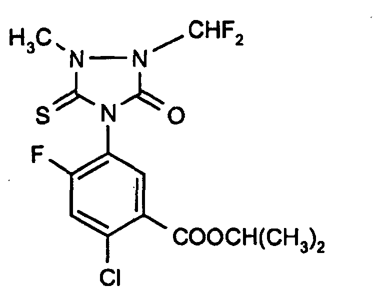

Eine Mischung aus 5,2 g (15 mMol) 4-(4-Chlor-2-fluor-5-i-propoxycarbonylphenyl)-1-methyl-5-thioxo-1,2,4-triazolin-3-on, 5,2 g (37,5 mMol) Kaliumcarbonat und 50 ml Acetonitril wird 60 Minuten bei 60°C gerührt; dann wird bei 60°C 6 Stunden lang Frigen (CHClF₂) eingeleitet. Anschließend wird unter vermindertem Druck eingeengt, der Rückstand in Wasser aufgenommen, mit konz. Salzsäure angesäuert, mit Dichlormethan extrahiert, die organische Phase über Natriumsulfat getrocknet und filtriert. Das Filtrat wird eingeengt und das im Rückstand erhaltene Rohprodukt durch Säulenchromatographie (Kieselgel, Hexan/Essigsäure-ethylester, Vol.: 7:1) gereinigt.

Man erhält 0,7 g (12% der Theorie) 4-(4-Chlor-2-fluor-5-i-propoxycarbonyl-phenyl)-1-methyl-2-difluormethyl-5-thioxo-1,2,4-triazolin-3-on vom Schmelzpunkt 51°C.

Analog zu Beispiel 1 sowie entsprechend der allgemeinen Beschreibung der erfindungsgemäßen Herstellungsverfahren können beispielsweise auch die in der nachstehenden Tabelle 1 aufgeführten Verbindungen der Formel (I) hergestellt werden.

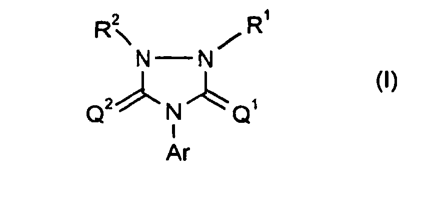

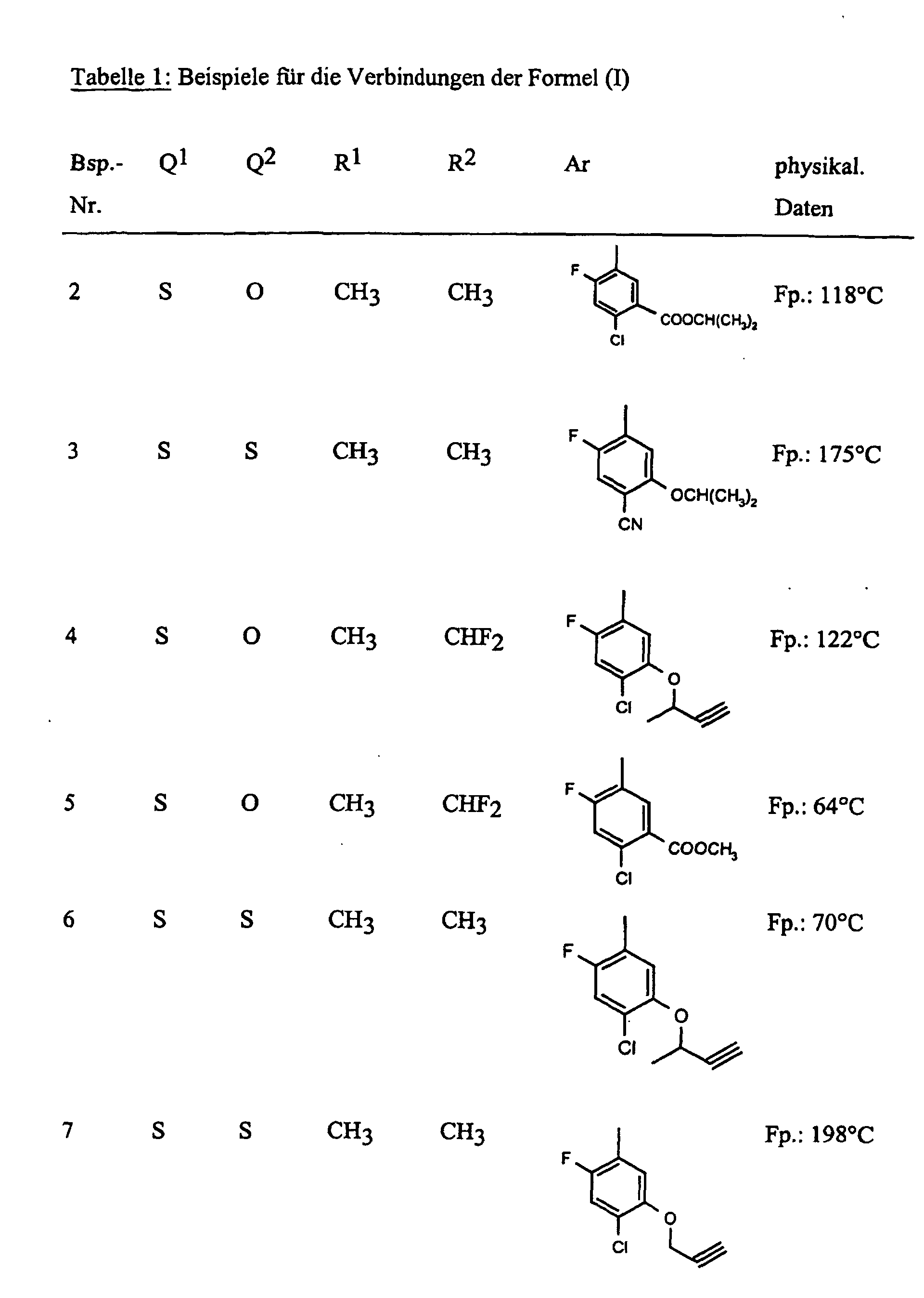

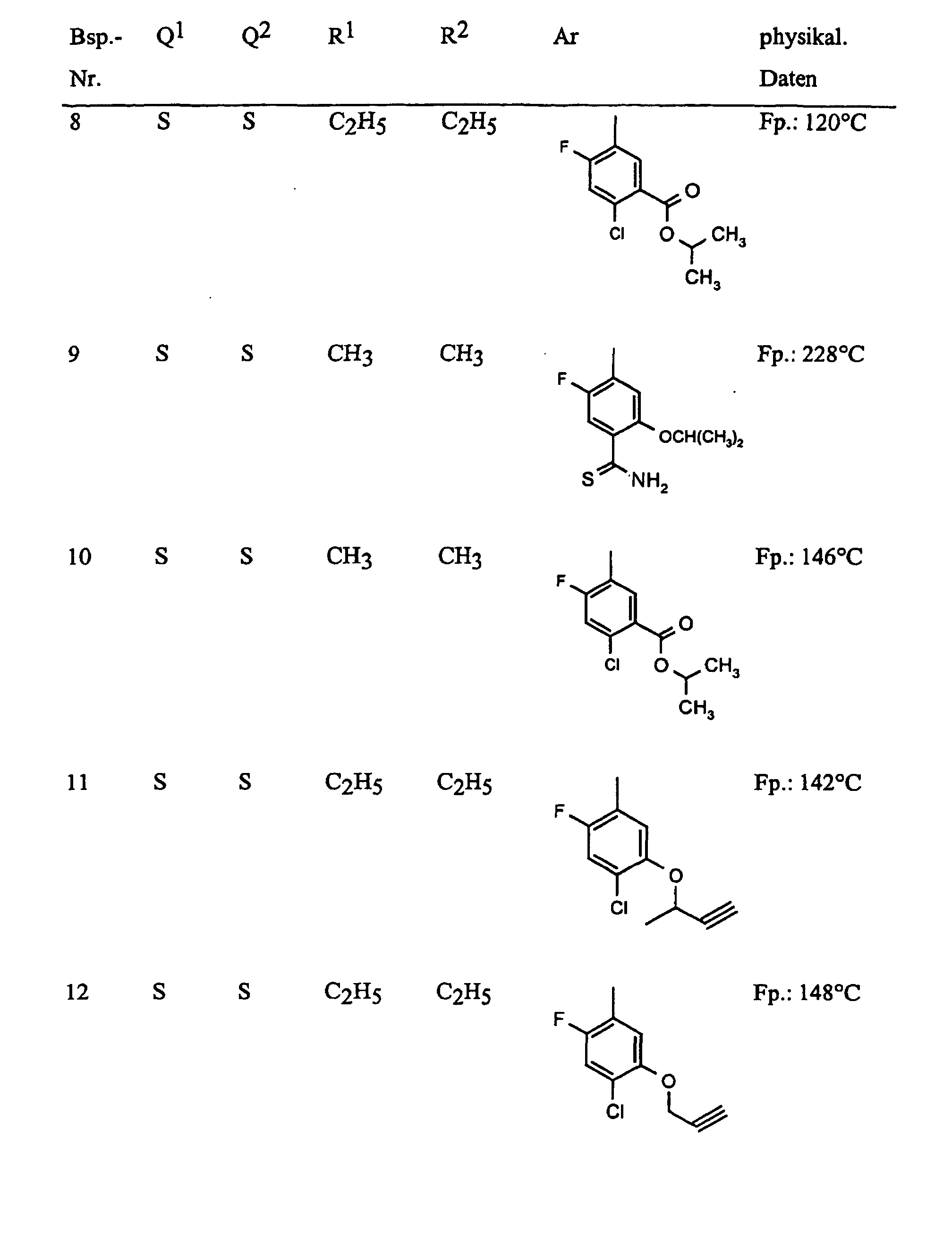

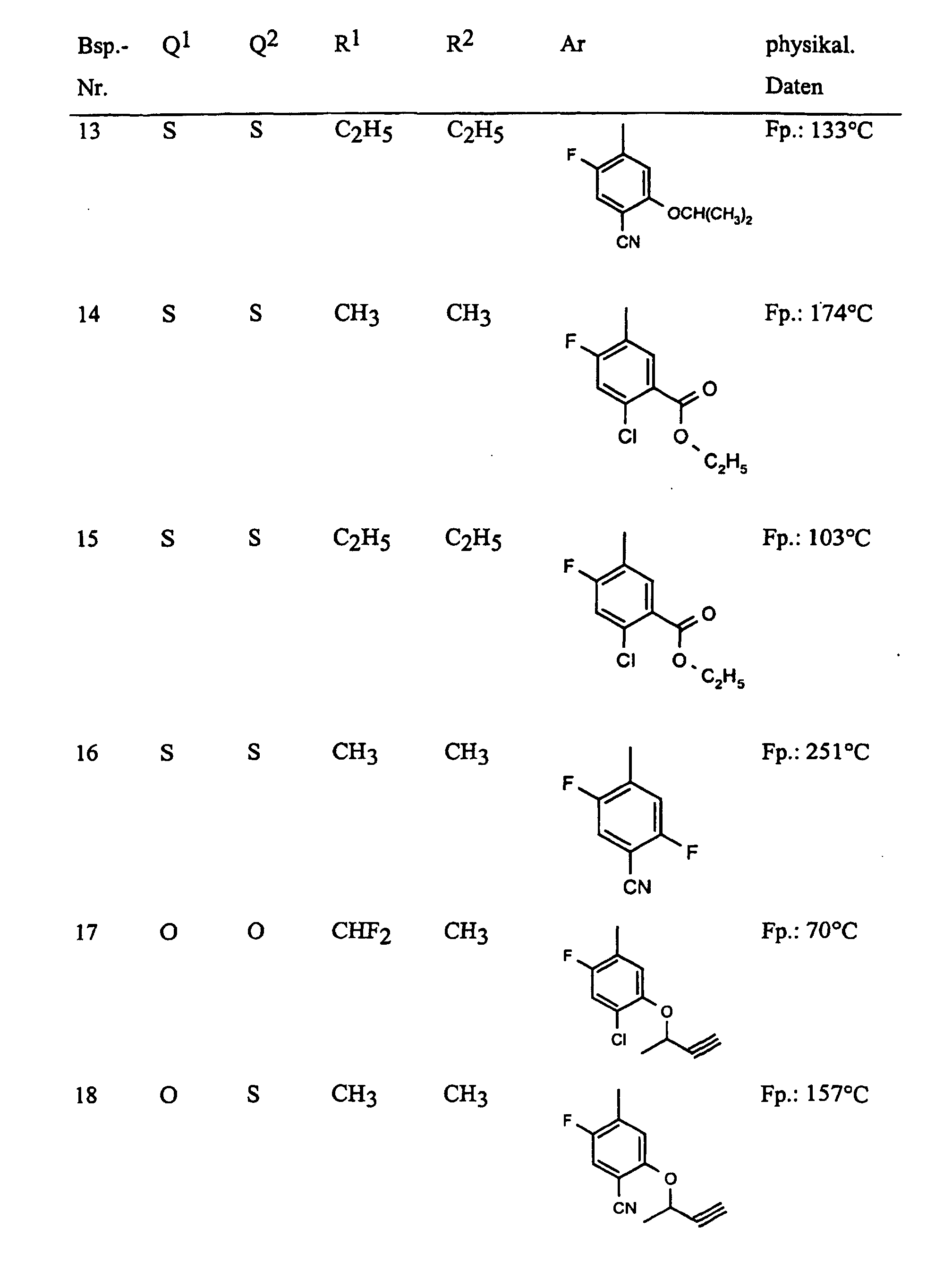

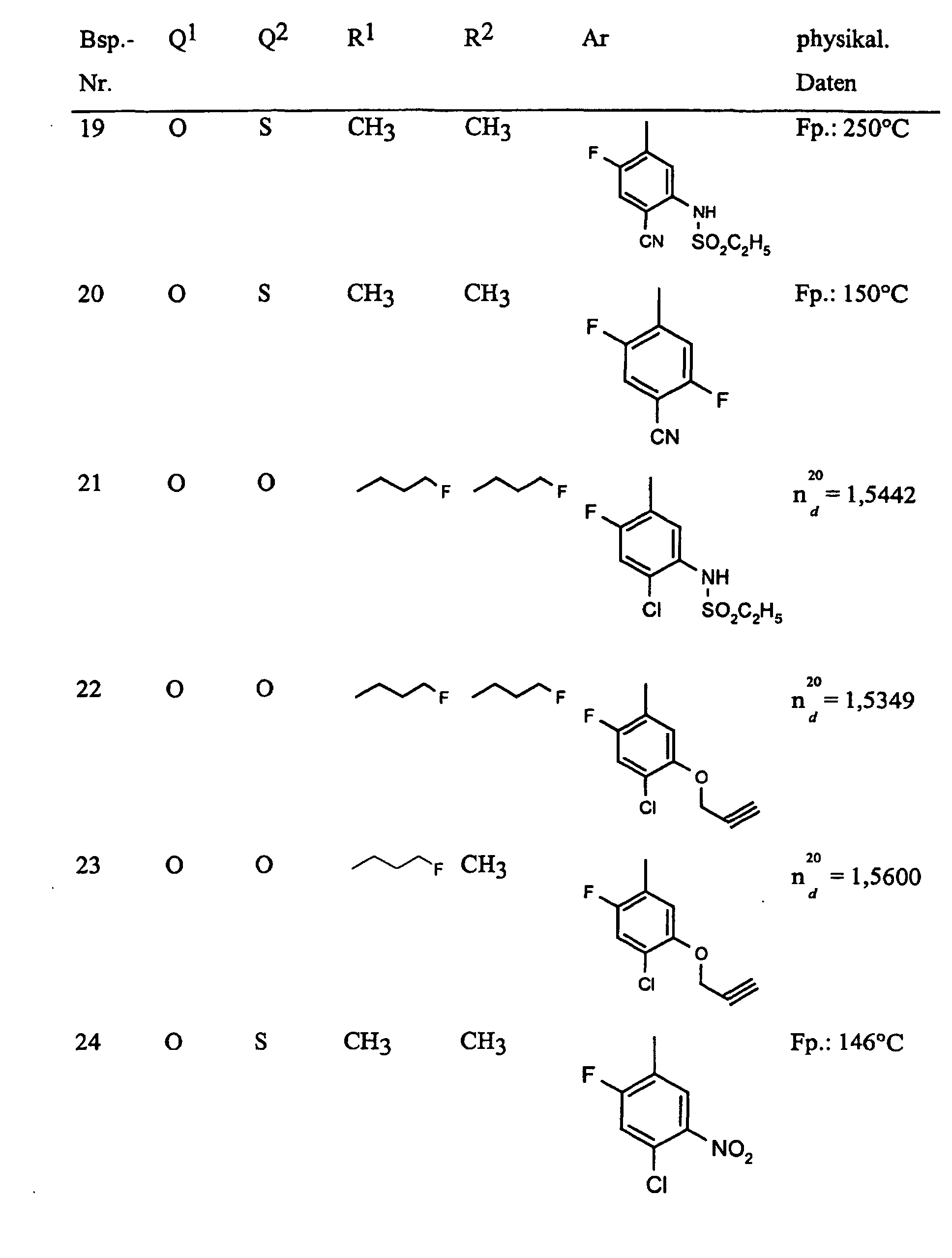

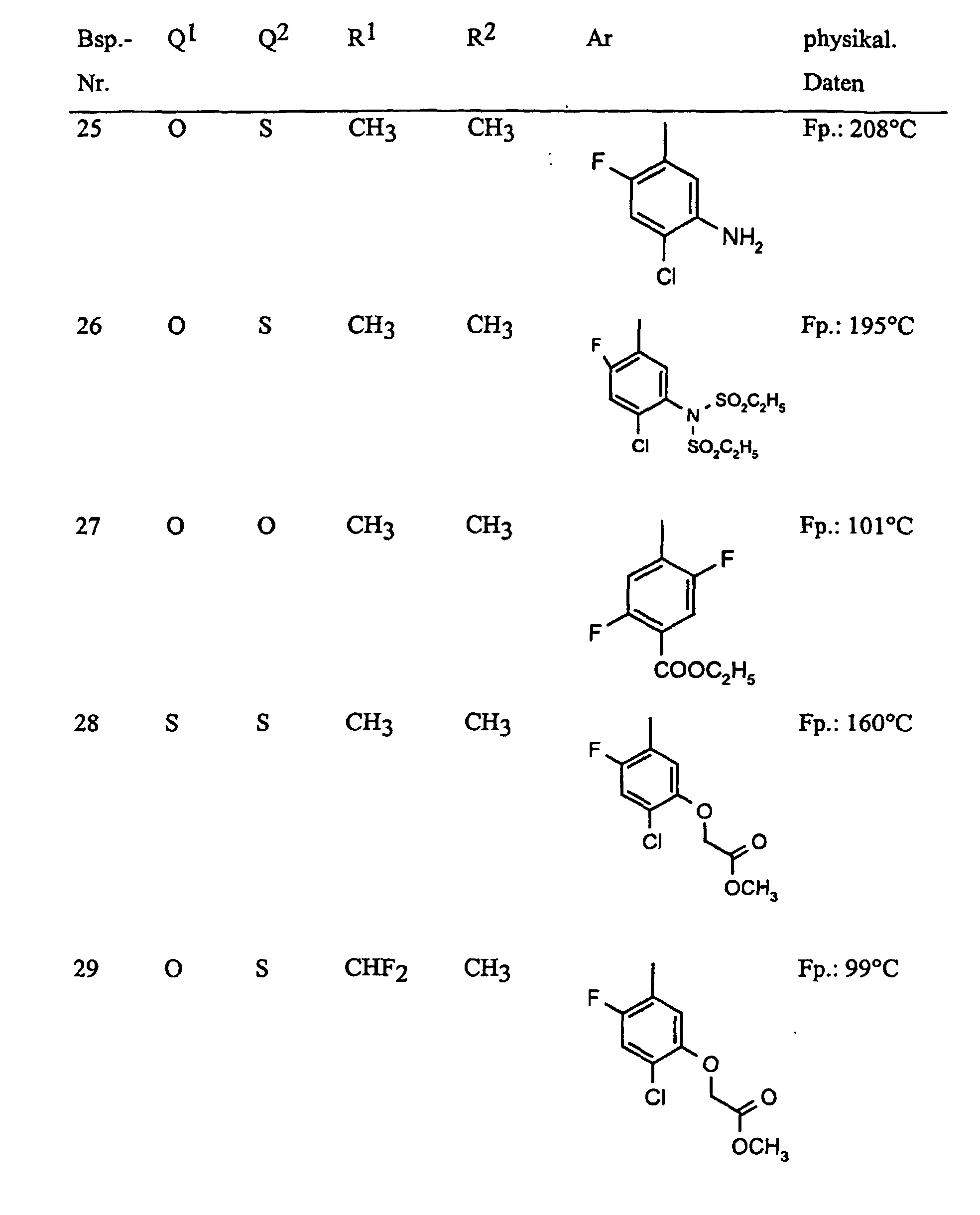

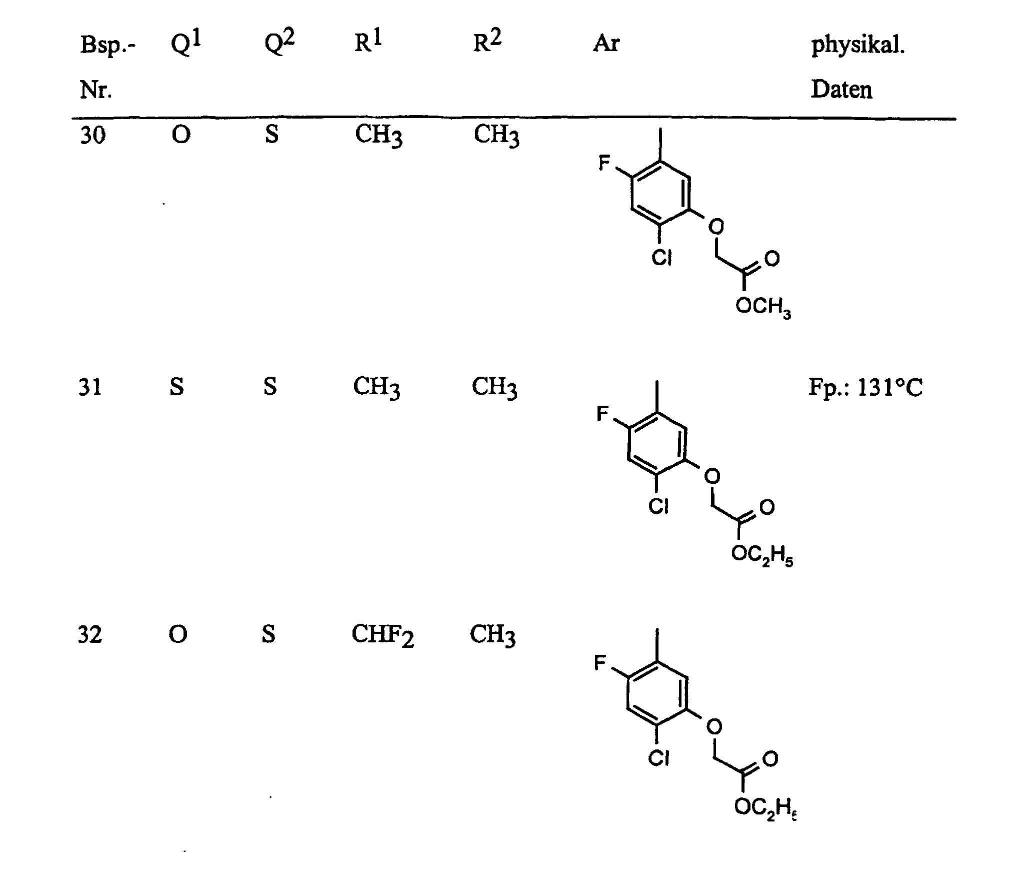

Die in Tabelle 1 als Beispiel 3 aufgeführte Verbindung kann beispielsweise wie folgt hergestellt werden:

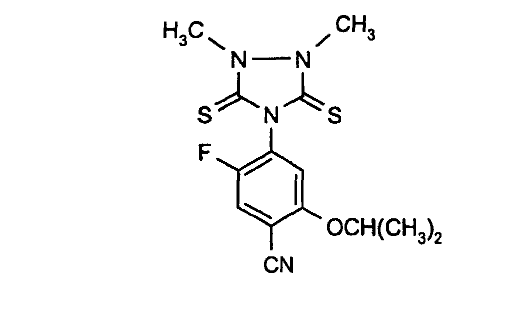

Eine Mischung aus 1,0 g (0,3 mMol) 2-(4-Cyano-2-fluor-4-i-propoxy-phenyl-imino)-3,4-dihydro-3,4-dimethyl-5-thioxo(4H)-1,3,4-thiadiazol, 0,1 g (0,1 mMol) Natriumsulfid und 20 ml Ethanol wird ca. 20 Stunden unter Rückfluß erhitzt. Dann wird eingeengt, der Rückstand in Dichlormethan aufgenommen, mit Wasser gewaschen, mit Natriumsulfat getrocknet und filtriert. Das Filtrat wird eingeengt, der Rückstand mit Diethylether digeriert und das kristallin anfallende Produkt durch Abfiltrieren isoliert.

Man erhält 0,25 g (25% der Theorie) 4-(4-Cyano-2-fluor-5-i-propoxy-phenyl)-1,2-dimethyl-1,2,4-triazolin-2,5-dithion vom Schmelzpunkt 175°C.

Die in Tabelle 1 als Beispiel 9 aufgeführte Verbindung kann beispielsweise wie folgt hergestellt werden:

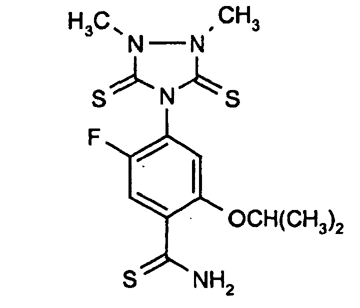

In eine Mischung aus 0,9 g (2,6 mMol) 4-(4-Cyano-2-fluor-5-i-propoxy-phenyl)-1,2-dimethyl-1,2,4-triazolin-2,5-dithion, 2 ml (14 mMol) Triethylamin und 20 ml Pyridin wird 9 Stunden bei 90°C Schwefelwasserstoff eingeleitet. Nach dem Abkühlen wird das Lösungsmittel am Rotationsverdampfer abgezogen und der Rückstand mit 2 normaler Salzsäure verrührt. Das Rohprodukt wird abgesaugt, mit Wasser gewaschen, getrocknet und durch Säulenchromatographie (Laufmittel: Hexan/Essigsäureethylester 4:1) gereinigt.

Man erhält 0,42 g (43 % der Theorie) 4-(2-Fluor-5-i-propoxy-4-thiocarbamoylphenyl)-1,2-dimethyl-1,2,4-triazolin-2,5-dithion vom Schmelzpunkt 228°C.

Die in Tabelle 1 als Beispiel 24 aufgeführte Verbindung kann beispielsweise wie folgt hergestellt werden:

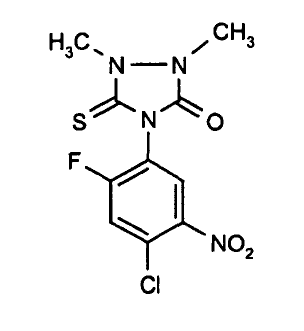

4,65 g (0,017 Mol) 4-(4-Chlor-2-fluor-phenyl)-1,2-dimethyl-5-thioxo-1,2,4-triazol-3-on werden in 30 ml konz. Schwefelsäure vorgelegt und bei 0°C tropfenweise mit 3 ml 98 %iger Salpetersäure versetzt. Man rührt 8 Stunden bei Raumtemperatur nach, verrührt mit Eiswasser und filtriert das ausgefallene Produkt ab.

Man erhält 2,9 g (54 % der Theorie) 4-(4-Chlor-2-fluor-5-nitro-phenyl)-1,2-dimethyl-5-thioxo-1,2,4-triazol-3-on vom Schmelzpunkt 146°C.

Die in Tabelle 1 als Beispiel 25 aufgeführte Verbindung kann beispielsweise wie folgt hergestellt werden:

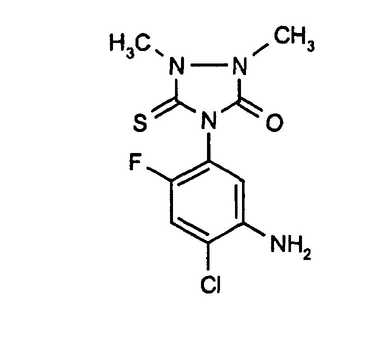

2,6 g (0,008 Mol) 4-(4-Chlor-2-fluor-5-nitro-phenyl)-1,2-dimethyl-5-thioxo-1,2,4-triazol-3-on werden in 20 ml Essigsäure mit 10 ml Wasser und 10 ml Essigsäureethylester vorgelegt und portionsweise mit 4,6 g (0,0082 Mol) Eisenpulver versetzt, wobei die Temperatur mit einem Eisbad bei max. 45°C gehalten wird. Nach beendeter Zugabe wird 2 Stunden bei 23°C nachgerührt, abgesaugt und der Rückstand mit Wasser gewaschen. Das Filtrat extrahiert man mit Essigsäureethylester, die organische Phase wird mit Natriumhydrogencarbonat-Lösung gewaschen, über Natriumsulfat getrocknet und im Wasserstrahlvakuum eingeengt.

Man erhält 1,5 g (65 % der Theorie) 4-(4-Chlor-2-fluor-5-amino-phenyl)-1,2-dimethyl-5-thioxo-1,2,4-triazol-3-on vom Schmelzpunkt 208°C.

Die in Tabelle 1 als Beispiel 26 aufgeführte Verbindung kann beispielsweise wie folgt hergestellt werden:

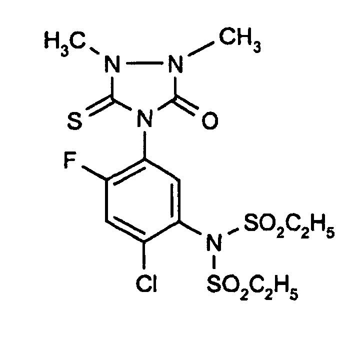

1,3 g (0,0045 Mol) 4-(4-Chlor-2-fluor-5-amino-phenyl)-1,2-dimethyl-5-thioxo-1,2,4-triazol-3-on werden in 50 ml Dichlormethan bei -10°C mit 1,8 g (0,018 Mol) Triethylamin und anschließend mit 2,3 g (0,018 Mol) Ethansulfonsäurechlorid versetzt, 3 Stunden bei Raumtemperatur gerührt, mit Wasser versetzt, die organische Phase abgetrennt, über Natriumsulfat getrocknet und im Wasserstrahlvakuum eingeengt.

Man erhält 2,0g (94 % der Theorie) 4-[4-Chlor-2-fluor-5-(diethylsulfonyl)-aminophenyl]-1,2-dimethyl-5-thioxo-1,2,4-triazol-3-on vom Schmelzpunkt 195°C (Zers.).

### Ausgangsstoffe der Formel (II):

### Beispiel (II-1)

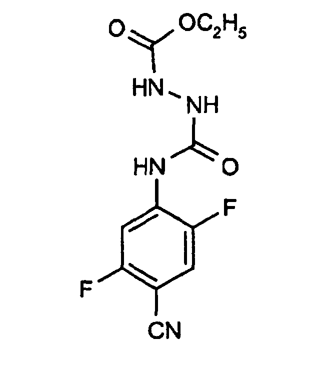

18,0 g (0,10 Mol) 4-Cyano-2,5-difluor-phenyl-isocyanat werden bei 10°C zu einer Lösung von 10,4 g (0,10 Mol) Ethylcarbazat in 100 ml Toluol gegeben und die Mischung wird 2 Stunden bei 20°C und dann weitere 2 Stunden bei Rückflußtemperatur gerührt. Das nach Abkühlen kristallin anfallende Produkt wird durch Abfiltrieren isoliert.

Man erhält 24,2 g (90 % der Theorie) 4-(4-Cyano-2,5-difluor-phenyl)-1-ethoxycarbonyl-semicarbazid vom Schmelzpunkt 245°C.

### Ausgangsstoffe der Formel (III):

### Beispiel (III-1)

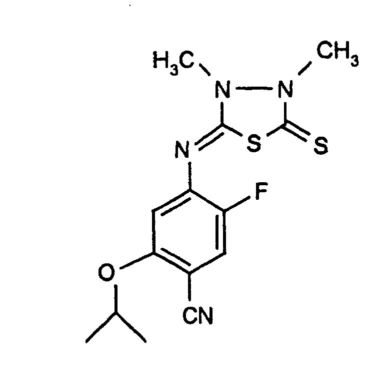

Zu einer Lösung von 1,7 g (5,7 mMol) 4-(4-Cyano-2-fluor-5-i-propoxy-phenyl)-1,2-dimethylthiosemicarbazid in 30 ml trockenem Dichlormethan gibt man 0,66 g (5,7 mMol) Thiophosgen. Die Reaktion ist leicht exotherm. Man rührt das Reaktionsgemisch 4 Stunden bei Rückflußtemperatur, entfernt das Lösungsmittel im Vakuum und verrührt den Rückstand mit gesättigter Natriumcarbonatlösung. Der sich bildende Feststoff wird abfiltriert, mit Wasser gewaschen und auf Ton abgepreßt.

Man erhält 1,5 g (78% der Theorie) 2-(4-Cyano-2-fluor-5-i-propoxy-phenylimino)-3,4-dimethyl-5-thio-1,3,4-thiadiazol vom Schmelzpunkt 117°C.

### Ausgangsstoffe der Formel (IV):

### Beispiel (IV-1)

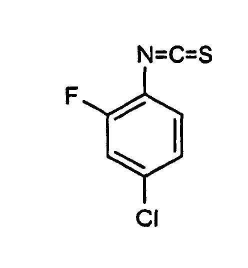

81 g (0,56 Mol) 4-Chlor-2-fluor-anilin werden in 500 ml Chlorbenzol bei 80°C bis 90°C innerhalb einer Stunde mit 129 g (1,12 Mol) Thiophosgen versetzt und anschließend bei Rückflußtemperatur 2 Stunden bis zur Beendigung der Gasentwicklung gerührt. Die klare Lösung wird im Wasserstrahlvakuum zur Trockene eingeengt.

Man erhält 102 g (97 % der Theorie) 4-Chlor-2-fluor-phenylisothiocyanat.
1H-NMR (CDCl₃): 7,10 - 7,20 ppm; GC-MS: (M=187) 98,4%ig

### Anwendungsbeispiele:

### Beispiel A

### Pre-emergence-Test

| | |
|---|---|
| Lösungsmittel | 5 Gewichtsteile Aceton |
| Emulgator | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:
0% = keine Wirkung (wie unbehandelte Kontrolle)
100% = totale Vernichtung

In diesem Test zeigen beispielsweise die Verbindungen gemäß den Herstellungsbeispielen 1, 2, 3, 4 und 5 bei Aufwandmengen von 60 g/ha teilweise gute Verträglichkeit gegenüber Kulturpflanzen, wie z.B. Gerste und Mais (10-70 %) und sehr starke Wirkung gegen Unkräuter wie Alopecurus (90-100 %), Cynodon (95 -100 %), Setaria (70-100 %), Amaranthus (90-100 %), Chenopodium (100 %), Matricaria (95-100%), Polygonum (80-100 %), Portulaca (95-100 %) und Viola (90-100 %).

### Beispiel B

### Post-emergence-Test

| | |
|---|---|
| Lösungsmittel | 5 Gewichtsteile Aceton |
| Emulgator | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben so, daß die jeweils gewünschten Wirkstoffmengen pro Flächeneinheit ausgebracht werden. Nach drei Wochen wird der Schädigungsgrad der Pflanzen bonitiert in % Schädigung im Vergleich zur Entwicklung der unbehandelten Kontrolle.

Es bedeuten:
0 % = keine Wirkung (wie unbehandelte Kontrolle) 100 % = totale Vernichtung

In diesem Test zeigen beispielsweise die Verbindungen gemäß den Herstellungsbeispielen 1, 2, 3, 4 und 5 bei Aufwandmengen zwischen 15 und 250 g/ha starke Wirkung gegen Unkräuter wie Amaranthus (60-100 %), Chenopodium (90-100 %), Datura (80-100 %), Galium (90-100 %) und Veronica (50-100 %).

## Patentansprüche

1. Substituierte N-Aryl-Stickstoffheterocyclen der allgemeinen Formel (I) 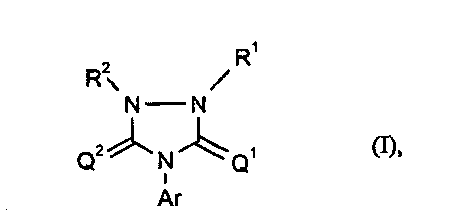 in welcher
Q¹ für Sauerstoff oder Schwefel steht,
Q² für Sauerstoff oder Schwefel steht,
R¹ für gegebenenfalls durch Fluor oder Chlor substituiertes C₁-C₆-Alkyl steht,
R² für gegebenenfalls durch Fluor oder Chlor substituiertes C₁-C₆-Alkyl steht,
Ar für die nachstehend definierte substituierte, monocyclische Aryl-Gruppierung steht, 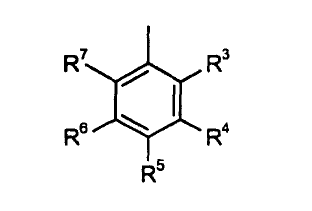 worin
R³ für Fluor, Chlor oder Brom steht,
R⁴ für Wasserstoff, Fluor, Chlor oder Brom steht,
R⁵ für Cyano, Carboxy, Chlorcarbonyl, Carbamoyl, Thiocarbamoyl, Hydroxy, Fluor, Chlor, Brom oder für jeweils gegebenenfalls durch Fluor und/oder Chlor substituiertes Alkyl, Alkoxy oder Alkoxycarbonyl mit jeweils bis zu 4 Kohlenstoffatomen steht,
R⁶ für die nachstehende Gruppierung steht,
- A¹-A²-A³
in welcher
A¹ für eine Einfachbindung oder für Sauerstoff, Schwefel, -SO-, -SO₂, -CO- oder die Gruppierung -N-A⁴- steht, worin A⁴ für Wasserstoff, Hydroxy, C₁-C₄-Alkyl, C₃-C₄-Alkenyl, C₃-C₄-Alkinyl, C₁-C₄-Alkoxy, Phenyl, C₁-C₄-Alkyl-carbonyl, Phenylcarbonyl, C₁-C₄-Alkyl-sulfonyl oder Phenylsulfonyl steht,
A¹ weiterhin für jeweils gegebenenfalls durch Fluor, Chlor oder Brom substituiertes C₁-C₆-Alkandiyl, C₂-C₆-Alkendiyl, C₂-C₆-Azaalkendiyl, C₂-C₆-Alkindiyl, C₃-C₆-Cycloalkandiyl, C₃-C₆-Cycloalkendiyl oder Phenylen steht,
A² für eine Einfachbindung oder für Sauerstoff, Schwefel, -SO-, -SO₂-, -CO- oder die Gruppierung -N-A⁴- steht, worin A⁴ für Wasserstoff, Hydroxy, C₁-C₄-Alkyl, C₁-C₄-Alkoxy, Phenyl, C₁-C₄-Alkylsulfonyl oder Phenylsulfonyl steht,
A² weiterhin für jeweils gegebenenfalls durch Fluor, Chlor oder Brom substituiertes C₁-C₆-Alkandiyl, C₂-C₆-Alkendiyl, C₂-C₆-Azaalkendiyl, C₂-C₆-Alkindiyl, C₃-C₆-Cycloalkandiyl, C₃-C₆-Cycloalkendiyl oder Phenylen steht,
A³ für Wasserstoff steht, mit der Maßgabe, dass in diesem Fall A¹ und/oder A² nicht für eine Einfachbindung stehen,
A³ weiterhin für Hydroxy, Mercapto, Amino, Cyano, Isocyano, Thiocyanato, Nitro, Carboxy, Carbamoyl, Thiocarbamoyl, Sulfo, Chlorsulfonyl, Fluor, Chlor, Brom steht,
A³ weiterhin für jeweils gegebenenfalls durch Fluor, Chlor oder C₁-C₄-Alkoxy substituiertes Alkyl, Alkoxy, Alkylthio, Alkylsulfinyl, Alkylsulfonyl, Alkylamino, Dialkylamino, Alkoxycarbonyl oder Dialkoxy-(thio)phosphoryl mit jeweils 1 bis 6 Kohlenstoffatomen in den Alkylgruppen steht,
A³ weiterhin für jeweils gegebenenfalls durch Fluor oder Chlor substituiertes Alkenyl, Alkenyloxy, Alkenylamino, Alkylidenamino, Alkenyloxycarbonyl, Alkinyl, Alkinyloxy, Alkinylamino oder Alkinyloxycarbonyl mit jeweils 2 bis 6 Kohlenstoffatomen in den Alkenyl-, Alkyliden- oder Alkinylgruppen steht,
A³ weiterhin für jeweils gegebenenfalls durch Fluor, Chlor, Cyano, Carboxy, C₁-C₄-Alkyl und/oder C₁-C₄-Alkoxy-carbonyl substituiertes Cycloalkyl; Cycloalkyloxy, Cycloalkylalkyl, Cycloalkylalkoxy, Cycloalkylidenamino, Cycloalkyloxycarbonyl oder Cycloalkylalkoxycarbonyl mit jeweils 3 bis 6 Kohlenstoffatomen in den Cycloalkylgruppen und gegebenenfalls 1 bis 4 Kohlenstoffatomen in den Alkylgruppen steht,
A³ weiterhin für jeweils gegebenenfalls durch Nitro, Cyano, Carboxy, Fluor, Chlor, Brom, C₁-C₄-Alkyl, C₁-C₄-Halogenalkyl, C₁-C₄-Alkyloxy, C₁-C₄-Halogenalkyloxy und/oder C₁-C₄-Alkoxy-carbonyl substituiertes Phenyl, Phenyloxy, Phenyl-C₁-C₄-alkyl, Phenyl-C₁-C₄-alkoxy, Phenyloxycarbonyl oder Phenyl-C₁-C₄-alkoxycarbonyl steht,
A³ weiterhin für jeweils gegebenenfalls ganz oder teilweise hydriertes Pyrrolyl, Pyrazolyl, Imidazolyl, Triazolyl, Furyl, Oxiranyl, Oxetanyl, Dioxolanyl, Thienyl, Oxazolyl, Isoxazolyl, Thiazolyl, Isothiazolyl, Oxadiazolyl, Thiadiazolyl, Pyridinyl, Pyrimidinyl, Triazinyl, Pyrazolyl-C₁-C₄-alkyl, Furyl-C₁-C₄-alkyl, Thienyl-C₁-C₄-alkyl, Oxazolyl-C₁-C₄-alkyl, Isoxazolyl-C₁-C₄-alkyl, Thiazolyl-C₁-C₄-alkyl, Pyridinyl-C₁-C₄-alkyl, Pyrimidinyl-C₁-C₄-alkyl, Pyrazolylmethoxy, Furylmethoxy, für Perhydropyranylmethoxy oder Pyridylmethoxy steht, und
R⁷ für Wasserstoff, Fluor oder Chlor steht.

2. Verbindungen gemäß Anspruch 1, **dadurch gekennzeichnet, dass**
Q¹ für Sauerstoff oder Schwefel steht,
Q² für Sauerstoff oder Schwefel steth,
R¹ für gegebenenfalls durch Fluor oder Chlor substituiertes Methyl, Ethyl, n- oder i-Propyl, n-, i-, s- oder t-Butyl steht,
R² für gegebenenfalls durch Fluor oder Chlor substituiertes Methyl, Ethyl, n- oder i-Propyl, n-, i-Propyl, n-, i-, s- oder t-Butyl steht,
Ar für die nachstehend definierte substituierte, monocyclische Aryl-Gruppierung steht, 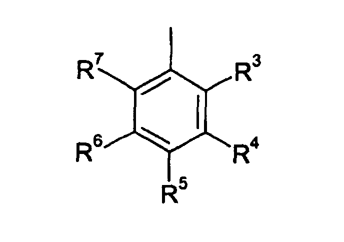 worin
R³ für Fluor oder Chlor steht,
R⁴ für Wasserstoff, Fluor oder Chlor steht,
R⁵ für Cyano, Thiocarbamoyl, Chlor, Brom, Methyl, Trifluormethyl, Methoxy, Difluormethoxy oder Trifluormethoxy steht,
R⁶ für die nachstehende Gruppierung steht,
-A¹-A²-A³
in welcher
A¹ für eine Einfachbindung oder für Sauerstoff, Schwefel, -SO-, -SO₂-, -CO- oder die Gruppierung -N-A⁴- steht, worin A⁴ für Wasserstoff, Hydroxy, Methyl, Ethyl, n- oder i-Propyl, Methoxy, Ethoxy, n- oder i-Propoxy, Methylsulfonyl oder Ethylsulfonyl steht,
A¹ weiterhin für Methylen, Ethan-1,1-diyl, Ethan-1,2-diyl, Propan-1,1-diyl, Propan-1,2-diyl, Propan-1,3-diyl, Ethen-1,2-diyl, Propen-1,2-diyl, Propen-1,3-diyl, Ethin-1,2-diyl, Propin-1,2-diyl oder Propin-1,3-diyl steht,
A² für eine Einfachbindung oder für Sauerstoff, Schwefel, -SO-, -SO₂-, -CO- oder die Gruppierung -N-A⁴- steht, worin A⁴ für Wasserstoff, Hydroxy, Methyl, Ethyl, n- oder i-Propyl, Methoxy, Ethoxy, n- oder i-Propoxy, Methylsulfonyl, Ethylsulfonyl, n- oder i-Propylsulfonyl oder Phenylsulfonyl steht,
A² weiterhin für Methylen, Ethan-1,1-diyl, Ethan-1,2-diyl, Propan-1,1-diyl, Propan-1,2-diyl, Propan-1,3-diyl, Ethen-1,2-diyl, Propen-1,2-diyl, Propen-1,3-diyl, Ethin-1,2-diyl, Propin-1,2-diyl oder Propin-1,3-diyl steht,
A³ für Wasserstoff steht, mit der Maßgabe, dass in diesem Fall A¹ und/oder A² nicht für eine Einfachbindung stehen,
A³ weiterhin für Hydroxy, Amino, Cyano, Nitro, Carboxy, Carbamoyl, Sulfo, Fluor, Chlor, Brom steht,
A³ weiterhin für jeweils gegebenenfalls durch Fluor, Chlor, Methoxy oder Ethoxy substituiertes Methyl, Ethyl, n- oder i-Propyl, n-, i-, s- oder t-Butyl, n-, i-, s- oder t-Pentyl, Methoxy, Ethoxy, n- oder i-Propoxy, n-, i-, s- oder t-Butoxy, n-, i-, s- oder t-Pentyloxy, Methylthio, Ethylthio, n- oder i-Propylthio, n-, i-, s- oder t-Butylthio, Methylsulfinyl, Ethylsulfinyl, n- oder i-Propylsulfinyl, Methylsulfonyl, Ethylsulfonyl, n-oder i-Propylsulfonyl, Methylamino, Ethylamino, n- oder i-Propylamino, n-, i-, s- oder t-Butylamino, Dimethylamino, Diethylamino, Methoxycarbonyl, Ethoxycarbonyl, n- oder i-Propoxycarbonyl, Dimethoxyphosphoryl, Diethoxyphosphoryl oder Dipropoxyphosphoryl, Diisopropoxyphosphoryl steht,
A³ weiterhin für jeweils gegebenenfalls durch Fluor oder Chlor substituiertes Propenyl, Butenyl, Propenyloxy, Butenyloxy, Propenylamino, Butenylamino, Propylidenamino, Butylidenamino, Propenyloxycarbonyl, Butenyloxycarbonyl, Propinyl, Butinyl, Propinyloxy, Butinyloxy, Propinylamino, Butinylamino, Propinyloxycarbonyl oder Butinyloxycarbonyl steht,
A³ weiterhin für jeweils gegebenenfalls durch Fluor, Chlor, Cyano, Carboxy, Methyl, Ethyl, n- oder i-Propyl, Methoxycarbonyl oder Ethoxycarbonyl substituiertes Cyclopropyl, Cyclobutyl, Cyclopentyl, Cyclohexyl, Cyclopropyloxy, Cyclobutyloxy, Cyclopentyloxy, Cyclohexyloxy, Cyclopropylmethyl, Cyclobutylmethyl, Cyclopentylmethyl, Cyclohexylmethyl, Cyclopropylmethoxy, Cyclobutylmethoxy, Cyclopentylmethoxy, Cyclohexylmethoxy, Cyclopentylidenamino, Cyclohexylidenamino, Cyclopentyloxycarbonyl, Cyclohexyloxycarbonyl, Cyclopentylmethoxycarbonyl oder Cyclohexylmethoxycarbonyl steht,
A³ weiterhin für jeweils gegebenenfalls durch Nitro, Cyano, Carboxy, Fluor, Chlor, Brom, Methyl, Ethyl, n- oder i-Propyl, Trifluormethyl, Methoxy, Ethoxy, n- oder i-Propoxy, Difluormethoxy, Trifluormethoxy, Methoxycarbonyl und/oder Ethoxycarbonyl substituiertes Phenyl, Phenyloxy, Benzyl, Phenylethyl, Benzyloxy, Phenyloxycarbonyl, Benzyloxycarbonyl steht,
A³ weiterhin für jeweils gegebenenfalls ganz oder teilweise hydriertes Pyrrolyl, Pyrazolyl, Imidazolyl, Triazolyl, Furyl, Thienyl, Oxazolyl, Isoxazolyl, Thiazolyl, Isothiazolyl, Oxadiazolyl, Thiadiazolyl, Pyridinyl, Pyrimidinyl, Triazinyl, Pyrazolylmethyl, Furylmethyl, Thienylmethyl, Oxazolylmethyl, Isoxazolylmethyl, Thiazolylmethyl, Pyridinylmethyl, Pyrimidinylmethyl, Pyrazolylmethoxy, Furylmethoxy oder Pyridylmethoxy steht,
R⁷ für Wasserstoff, Fluor oder Chlor steht.

3. Verfahren zur Herstellung von Verbindungen gemäß Anspruch 1 oder 2, **dadurch gekennzeichnet, dass** man
(a) (Thio)Semicarbazid-Derivate der allgemeinen Formel (II) 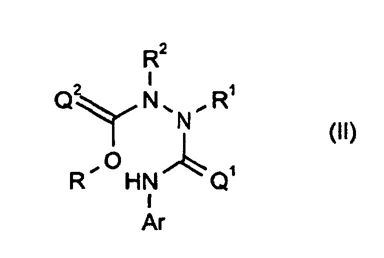 in welcher
Q¹, Q², R¹, R² und Ar die in Anspruch 1 angegebenen Bedeutungen haben und
R für Alkyl steht,
gegebenenfalls in Gegenwart eines Reaktionshilfsmittels und gegebenenfalls in Gegenwart eines Verdünnungsmittels cyclisierend kondensiert und gegebenenfalls im Anschluss daran im Rahmen der Substituentendefinition auf übliche Weise elektrophile oder nucleophile Substitutionsreaktionen durchführt,
oder dass man
(b) Aryliminoheterocyclen der allgemeinen Formel (III) 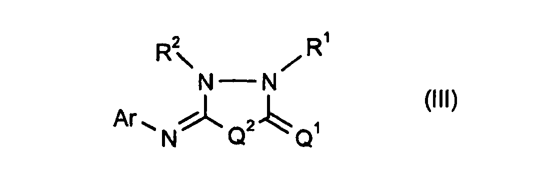 in welcher
Q¹, Q², R¹, R² und Ar die oben angegebenen Bedeutungen haben,
gegebenenfalls in Gegenwart eines Reaktionshilfsmittels und gegebenenfalls in Gegenwart eines Verdünnungsmittels thermisch ("pyrolytisch") isomerisiert.

4. Herbizide Mittel, **gekennzeichnet durch** einen Gehalt an mindestens einer Verbindung gemäß Anspruch 1 oder 2.

5. Verfahren zur Bekämpfung von unerwünschten Pflanzen, **dadurch gekennzeichnet, dass** man eine Verbindung gemäß Anspruch 1 oder 2 auf unerwünschte Pflanzen und/oder ihren Lebensraum einwirken lässt.

6. Verwendung einer Verbindung gemäß Anspruch 1 oder 2 zur Bekämpfung von unerwünschten Pflanzen.

7. Verfahren zur Herstellung von herbiziden Mitteln, **dadurch gekennzeichnet, dass** man eine Verbindung gemäß Anspruch 1 oder 2 mit Streckmitteln und/oder oberflächenaktiven Substanzen vermischt.

## Claims

1. Substituted N-aryl nitrogen-containing heterocyclic compounds of the general formula (I) 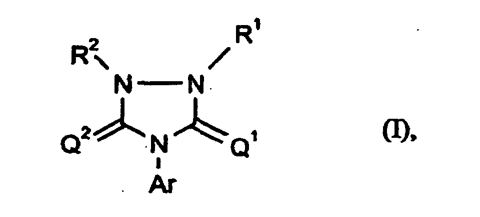 in which
Q¹ represents oxygen or sulfur,
Q² represents oxygen or sulfur,
R¹ represents C₁-C₆-alkyl which is optionally substituted by fluorine or chlorine,
R² represents C₁-C₆-alkyl which is optionally substituted by fluorine or chlorine,
Ar represents the substituted monocyclic aryl grouping defined below, 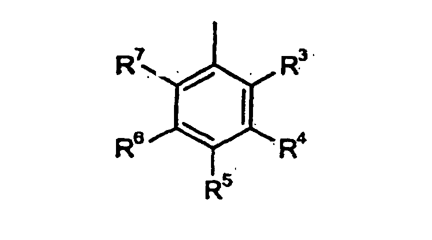 in which
R³ represents fluorine, chlorine or bromine,
R⁴ represents hydrogen, fluorine, chlorine or bromine,
R⁵ represents cyano, carboxyl, chlorocarbonyl, carbamoyl, thiocarbamoyl, hydroxyl, fluorine, chlorine, bromine or represents alkyl, alkoxy or alkoxycarbonyl having in each case up to 4 carbon atoms and in each case optionally substituted by fluorine and/or chlorine,
R⁶ represents the following grouping
-A¹-A²-A³
in which
A¹ represents a single bond, or represents oxygen, sulfur, -SO-, -SO₂-, -CO- or the grouping -N-A⁴-, in which A⁴ represents hydrogen, hydroxyl, C₁-C₄-alkyl, C₃-C₄-alkenyl, C₃-C₄-alkinyl, C₁-C₄-alkoxy, phenyl, C₁-C₄-alkyl-carbonyl, phenylcarbonyl, C₁-C₄-alkyl-sulfonyl or phenylsulfonyl,
A¹ furthermore represents C₁-C₆-alkanediyl, C₂-C₆-alkenediyl, C₂-C₆-azaalkenediyl, C₂-C₆-alkinediyl, C₃-C₆-cycloalkanediyl, C₃-C₆-cycloalkenediyl or phenylene, in each case optionally substituted by fluorine, chlorine or bromine,
A² represents a single bond, or represents oxygen, sulfur, -SO-, -SO₂-, -CO- or the grouping -N-A⁴-, in which A⁴ represents hydrogen, hydroxyl, C₁-C₄-alkyl, C₁-C₄-alkoxy, phenyl, C₁-C₄-alkylsulfonyl or phenylsulfonyl,
A² furthermore represents C₁-C₆-alkanediyl, C₂-C₆-alkenediyl, C₂-C₆-azaalkenediyl, C₂-C₆-alkinediyl, C₃-C₆-cycloalkanediyl, C₃-C₆-cycloalkenediyl or phenylene, in each case optionally substituted by fluorine, chlorine or bromine,
A³ represents hydrogen, with the proviso that in this case A¹ and/or A² do(es) not represent a single bond,
A³ furthermore represents hydroxyl, mercapto, amino, cyano, isocyano, thiocyanato, nitro, carboxyl, carbamoyl, thiocarbamoyl, sulfo, chlorosulfonyl, fluorine, chlorine or bromine,
A³ furthermore represents alkyl, alkoxy, alkylthio, alkylsulfinyl, alkylsulfonyl, alkylamino, dialkylamino, alkoxycarbonyl or dialkoxy(thio)phosphoryl having in each case 1 to 6 carbon atoms in the alkyl groups and in each case optionally substituted by fluorine, chlorine or C₁-C₄-alkoxy,
A³ furthermore represents alkenyl, alkenyloxy, alkenylamino, alkylidenamino, alkenyloxycarbonyl, alkinyl, alkinyloxy, alkinylamino or alkinyloxycarbonyl having in each case 2 to 6 carbon atoms in the alkenyl, alkylidene or alkinyl groups and in each case optionally substituted by fluorine or chlorine,
A³ furthermore represents cycloalkyl cycloalkyloxy, cycloalkylalkyl, cycloalkylalkoxy, cycloalkylidenamino, cycloalkyloxycarbonyl or cycloalkylalkoxycarbonyl having in each case 3 to 6 carbon atoms in the cycloalkyl groups and where appropriate 1 to 4 carbon atoms in the alkyl groups and in each case optionally substituted by fluorine, chlorine, cyano, carboxyl, C₁-C₄-alkyl and/or C₁-C₄-alkoxy-carbonyl,
A³ furthermore represents phenyl, phenyloxy, phenyl-C₁-C₄-alkyl, phenyl-C₁-C₄-alkoxy, phenyloxycarbonyl or phenyl-C₁-C₄-alkoxycarbonyl, in each case optionally substituted by nitro, cyano, carboxyl, fluorine, chlorine, bromine, C₁-C₄-alkyl, C₁-C₄-halogenalkyl, C₁-C₄-alkyloxy, C₁-C₄-halogenalkyloxy and/or C₁-C₄-alkoxy-carbonyl,
A³ furthermore represents in each case optionally completely or partly hydrogenated pyrrolyl, pyrazolyl, imidazolyl, triazolyl, furyl, oxiranyl, oxetanyl, dioxolanyl, thienyl, oxazolyl, isoxazolyl, thiazolyl, isothiazolyl, oxadiazolyl, thiadiazolyl, pyridinyl, pyrimidinyl, triazinyl, pyrazolyl-C₁-C₄-alkyl, furyl-C₁-C₄-alkyl, thienyl-C₁-C₄-alkyl, oxalolyl-C₁-C₄-alkyl, isoxazolyl-C₁-C₄-alkyl, thiazolyl-C₁-C₄-alkyl, pyridinyl-C₁-C₄-alkyl, pyrimidinyl-C₁-C₄-alkyl, pyrazolylmethoxy or furylmethoxy, or represents perhydropyranylmethoxy or pyridylmethoxy, and
R⁷ represents hydrogen, fluorine or chlorine.

2. Compounds according to Claim 1, **characterized in that**
Q¹ represents oxygen or sulfur,
Q² represents oxygen or sulfur,
R¹ represents methyl, ethyl, n- or i-propyl or n-, i-, s- or t-butyl which is optionally substituted by fluorine or chlorine,
R² represents methyl, ethyl, n- or i-propyl, n- or i-propyl or n-, i-, s- or t-butyl which is optionally substituted by fluorine or chlorine,
Ar represents the substituted monocyclic aryl grouping defined below 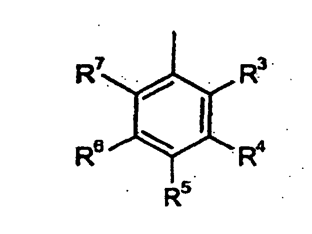 in which
R³ represents fluorine or chlorine,
R⁴ represents hydrogen, fluorine or chlorine,
R⁵ represents cyano, thiocarbamoyl, chlorine, bromine, methyl, trifluoromethyl, methoxy, difluoromethoxy or trifluoromethoxy,
R⁶ represents the following grouping
-A¹-A²-A³
in which
A¹ represents a single bond, or represents oxygen, sulfur, -SO-, -SO₂-, -CO- or the grouping -N-A⁴-, in which A⁴ represents hydrogen, hydroxyl, methyl, ethyl, n- or i-propyl, methoxy, ethoxy, n- or i-propoxy, methylsulfonyl or ethylsulfonyl,
A¹ furthermore represents methylene, ethane-1,1-diyl, ethane-1,2-diyl, propane-1,1-diyl, propane-1,2-diyl, propane-1,3-diyl, ethene-1,2,-diyl, propene-1,2-diyl, propene-1,3-diyl, ethine-1,2-diyl, propine-1,2-diyl or propine-1,3-diyl,
A² represents a single bond, or represents oxygen, sulfur, -SO-, -SO₂-, -CO- or the grouping -N-A⁴-, in which A⁴ represents oxygen, hydroxyl, methyl, ethyl, n- or i-propyl, methoxy, ethoxy, n- or i-propoxy, methylsulfonyl, ethylsulfonyl, n- or i-propylsulfonyl or phenylsulfonyl,
A² furthermore represents methylene, ethane-1,1-diyl, ethane-1,2-diyl, propane-1,1-diyl, propane-1,2-diyl, propane-1,3-diyl, ethene-1,2-diyl, propene-1,2-diyl, propene-1,3-diyl, ethine-1,2-diyl, propine-1,2-diyl or propine-1,3-diyl,
A³ represents hydrogen, with the proviso that in this case A¹ and/or A² do(es) not represent a single bond,
A³ furthermore represents hydroxyl, amino, cyano, nitro, carboxyl, carbamoyl, sulfo, fluorine, chlorine or bromine,
A³ furthermore represents methyl, ethyl, n- or i-propyl, n-, i-, s- or t-butyl, n-, i-, s- or t-pentyl, methoxy, ethoxy, n- or i-propoxy, n-, i-, s- or t-butoxy, n-, i-, s- or t-pentyloxy, methylthio, ethylthio, n- or i-propylthio, n-, i-, s- or t-butylthio, methylsulfinyl, ethylsulfinyl, n- or i-propylsulfinyl, methylsulfonyl, ethylsulfonyl, n- or i- propylsulfonyl, methylamino, ethylamino, n- or i-propylamino, n-, i-, s- or t-butylamino, dimethylamino, diethylamino, methoxycarbonyl, ethoxycarbonyl, n- or i-propoxycarbonyl, dimethoxyphosphoryl, diethoxyphosphoryl, dipropoxyphosphoryl or diisopropoxyphosphoryl, in each case optionally substituted by fluorine, chlorine, methoxy or ethoxy,
A³ furthermore represents propenyl, butenyl, propenyloxy, butenyloxy, propenylamino, butenylamino, propylidenamino, butylidenamino, propenyloxycarbonyl, butenyloxycarbonyl, propinyl, butinyl, propinyloxy, butinyloxy, propinylamino, butinylamino, propinyloxycarbonyl or butinyloxycarbonyl, in each case optionally substituted by fluorine or chlorine,
A³ furthermore represents cyclopropyl, cyclobutyl, cyclopentyl, cyclohexyl, cyclopropyloxy, cyclobutyloxy, cyclopentyloxy, cyclohexyloxy, cyclopropylmethyl, cyclobutylmethyl, cyclopentylmethyl, cyclohexylmethyl, cyclopropylmethoxy, cyclobutylmethoxy, cyclopentylmethoxy, cyclohexylmethoxy, cyclopentylidenamino, cyclohexylidenamino, cyclopentyloxycarbonyl, cyclohexyloxycarbonyl, cyclopentylmethoxycarbonyl or cyclohexylmethoxycarbonyl, in each case optionally substituted by fluorine, chlorine, cyano, carboxyl, methyl, ethyl, n- or i-propyl, methoxycarbonyl or ethoxycarbonyl,
A³ furthermore represents phenyl, phenyloxy, benzyl, phenylethyl, benzyloxy, phenyloxycarbonyl or benzyloxycarbonyl, in each case optionally substituted by nitro, cyano, carboxyl, fluorine, chlorine, bromine, methyl, ethyl, n- or i-propyl, trifluoromethyl, methoxy, ethoxy, n- or i-propoxy, difluoromethoxy, trifluoromethoxy, methoxycarbonyl and/or ethoxycarbonyl,
A³ furthermore represents in each case optionally completely or partly hydrogenated pyrrolyl, pyrazolyl, imidazolyl, triazolyl, furyl, thienyl, oxazolyl, isoxazolyl, thiazolyl, isothiazolyl, oxadiazolyl, thiadiazolyl, pyridinyl, pyrimidinyl, triazinyl, pyrazolylmethyl, furylmethyl, thienylmethyl, oxazolylmethyl, isoxazolylmethyl, thiazolylmethyl, pyridinylmethyl, pyrimidinylmethyl, pyrazolylmethoxy, furylmethoxy or pyridylmethoxy,
R⁷ represents hydrogen, fluorine or chlorine.

3. Process for the preparation of compounds according to Claim 1 or 2, **characterized in that**
(a) (thio)semicarbazide derivatives of the general formula (II) 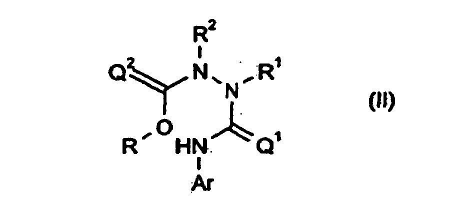 in which
Q¹, Q², R¹, R² and Ar have the meanings given in claim 1 and
R represents alkyl,
are subjected to a cyclizing condensation reaction, if appropriate in the presence of a reaction auxiliary and if appropriate in the presence of a diluent, and thereafter, if appropriate, electrophilic or nucleophilic substitution reactions are carried out in the customary manner in the context of the definition of the substituents,
or **in that**
(b) aryliminoheterocyclic compounds of the general formula (III) 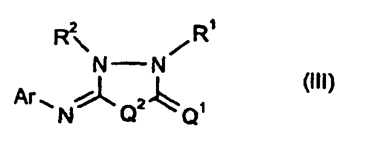 in which
Q¹, Q², R¹, R² and Ar have the abovementioned meanings,
are isomerized thermally ("pyrolytically"), if appropriate in the presence of a reaction auxiliary and if appropriate in the presence of a diluent.

4. Herbicidal composition **characterized by** a content of at least one compound according to Claim 1 or 2.

5. Method of combating undesirable plants, **characterized in that** a compound according to Claim 1 or 2 is allowed to act on undesirable plants and/or their environment.

6. Use of a compound according to Claim 1 or 2 for combating undesirable plants.

7. Process for the preparation of herbicidal compositions, **characterized in that** a compound according to Claim 1 or 2 is mixed with extenders and/or surface-active substances.

## Revendications

1. N-Arylhétérocycles azotés substitués de la formule générale (I) : 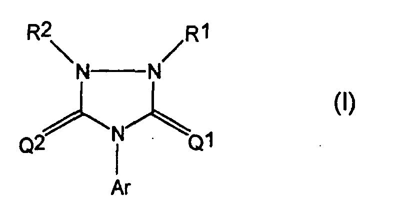 dans laquelle :
Q¹ représente l'atome d'oxygène ou de soufre,
Q² représente l'atome d'oxygène ou de soufre,
R¹ représente un radical alcoyle en C₁-C₆ le cas échéant substitué par l'atome de fluor ou de chlore,
R² représente un radical alcoyle en C₁-C₆ le cas échéant substitué par l'atome de fluor ou de chlore,
Ar représente le groupement aryle monocyclique, substitué, défini ci-après : 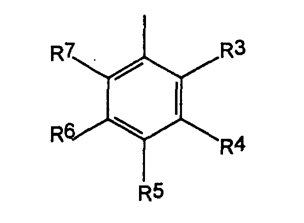
où
R³ représente l'atome de fluor, de chlore ou de brome,
R⁴ représente l'atome d'hydrogène, de fluor, de chlore ou de brome,
R⁵ représente le radical cyano, carboxy, chlorocarbonyle, carbamoyle, thiocarbamoyle, hydroxy, l'atome de fluor, de chlore, de brome, ou représente un radical alcoyle, alcoxy ou alcoxycarbonyle, avec chaque fois, jusqu'à 4 atomes de carbone, chaque fois le cas échéant substitué par l'atome de fluor et/ou de chlore,
R⁶ représente le groupement suivant :
-A¹-A²-A³
dans lequel :
A¹ représente une simple liaison ou l'atome d'oxygène, de soufre, le radical -SO-, -SO₂-, -CO- ou le groupement -N-A⁴-, où A⁴ représente l'atome d'hydrogène, le radical hydroxy, un radical alcoyle en C₁-C₄, alcényle en C₃-C₄, alcynyle en C₃-C₄, alcoxy en C₁-C₄, phényle, (alcoyl en C₁-C₄)carbonyle, phénylcarbonyle, (alcoyl en C₁-C₄)sulfonyle, phénylsulfonyle,
A¹ représente par ailleurs, un radical alcanediyle en C₁-C₆, alcènediyle en C₂-C₆, azaalcènediyle en C₂-C₆, alcynediyle en C₂-C₆, cycloalcanediyle en C₃-C₆, cycloalcènediyle en C₃-C₆ ou phénylène, chaque fois le cas échéant substitué par l'atome de fluor, de chlore ou de brome,
A² représente une simple liaison ou l'atome d'oxygène, de soufre, le radical -SO-, -SO₂-, -CO- ou le groupement -N-A⁴-, où A⁴ représente l'atome d'hydrogène, le radical hydroxy, un radical alcoyle en C₁-C₄, alcoxy en C₁-C₄, phényle, (alcoyl en C₁-C₄)sulfonyle ou phénylsulfonyle,
A² représente par ailleurs, un radical alcanediyle en C₁-C₆, alcènediyle en C₂-C₆, azaalcènediyle en C₂-C₆, alcynediyle en C₂-C₆, cycloalcanediyle en C₃-C₆, cycloalcènediyle en C₃-C₆ ou phénylène, chaque fois le cas échéant substitué par l'atome de fluor, de chlore ou de brome,
A³ représente l'atome d'hydrogène, avec la condition que dans ce cas, A¹ et/ou A² ne représentent pas une simple liaison,
A³ représente par ailleurs, le radical hydroxy, mercapto, amino, cyano, isocyano, thiocyanato, nitro, carboxy, carbamoyle, thiocarbamoyle, sulfo, chlorosulfonyle, l'atome de fluor, de chlore, de brome,
A³ représente par ailleurs, un radical alcoyle, alcoxy, alcoylthio, alcoylsulfinyle, alcoylsulfonyle, alcoylamino, dialcoylamino, alcoxycarbonyle ou dialcoxy(thio)phosphoryle avec chaque fois, 1 à 6 atomes de carbone dans les radicaux alcoyle, chaque fois le cas échéant substitué par l'atome de fluor, de chlore ou le radical alcoxy en C₁-C₄,
A³ représente par ailleurs, un radical alcényle, alcényloxy, alcénylamino, alcoylidèneamino, alcényloxycarbonyle, alcynyle, alcynyloxy, alcynylamino ou alcynyloxycarbonyle avec chaque fois, 2 à 6 atomes de carbone dans les radicaux alcényle, alcoylidène ou alcynyle, chaque fois le cas échéant substitué par l'atome de fluor ou de chlore,
A³ représente par ailleurs, un radical cycloalcoyle, cycloalcoxy, cycloalcoylalcoyle, cycloalcoylalcoxy, cycloalcoylidèneamino, cycloalcoyloxycarbonyle ou cycloalcoylalcoxycarbonyle, avec chaque fois, 3 à 6 atomes de carbone dans les radicaux cycloalcoyle, et le cas échéant, 1 à 4 atomes de carbone dans les radicaux alcoyle, chaque fois le cas échéant substitué par l'atome de fluor, de chlore, le radical cyano, carboxy, un radical alcoyle en C₁-C₄ ou (alcoxy en C₁-C₄)carbonyle,
A³ représente par ailleurs, un radical phényle, phényloxy, phényl(alcoyle en C₁-C₄), phényl(alcoxy en C₁-C₄), phényloxycarbonyle ou phényl(alcoxy en C₁-C₄)carbonyle, chaque fois le cas échéant substitué par le radical nitro, cyano, carboxy, l'atome de fluor, de chlore, de brome, un radical alcoyle en C₁-C₄, halogénoalcoyle en C₁-C₄ et/ou (alcoxy en C₁-C₄)carbonyle,
A³ représente par ailleurs, un radical pyrrolyle, pyrazolyle, imidazolyle, triazolyle, furyle, oxirannyle, oxétannyle, dioxolannyle, thiényle, oxazolyle, isoxazolyle, thiazolyle, isothiazolyle, oxadiazoylyle, thiadiazolyle, pyridinyle, pyrimidinyle, triazinyle, pyrazolyl (alcoyle en C₁-C₄), furyl(alcoyle en C₁-C₄), thiényl(alcoyle en C₁-C₄), oxazolyl(alcoyle en C₁-C₄), isoxazolyl (alcoyle en C₁-C₄), thiazolyl(alcoyle en C₁-C₄), pyridinyl(alcoyle en C₁-C₄), pyrimidinyl(alcoyle en C₁-C₄), pyrazolylméthoxy, furylméthoxy, chaque fois le cas échéant complètement ou partiellement hydrogéné, représente le radical perhydropyrannylméthoxy ou pyridylméthoxy, et
R⁷ représente l'atome d'hydrogène, de fluor ou de chlore.

2. Composés selon la revendication 1, **caractérisés en ce que**
Q¹ représente l'atome d'oxygène ou de soufre,
Q² représente l'atome d'oxygène ou de soufre,
R¹ représente le radical méthyle, éthyle, n- ou i-propyle, n-, i-, s- ou t-butyle, le cas échéant substitué par l'atome de fluor ou de chlore,
R² représente le radical méthyle, éthyle, n- ou i-propyle, n-, i-, s- ou t-butyle, le cas échéant substitué par l'atome de fluor ou de chlore,
Ar représente le groupement aryle monocyclique, substitué, défini ci-après : 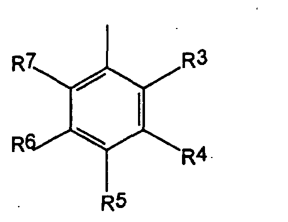 où
R³ représente l'atome de fluor ou de chlore,
R⁴ représente l'atome d'hydrogène, de fluor ou de chlore,
R⁵ représente le radical cyano, thiocarbamoyle, l'atome de chlore, de brome, le radical trifluorométhyle, méthoxy, difluorométhoxy ou trifluorométhoxy,
R⁶ représente le groupement suivant :
-A¹-A²-A³
dans lequel :
A¹ représente une simple liaison ou l'atome d'oxygène, de soufre, le radical -SO-, -SO₂-, -CO- ou le groupement -N-A⁴-, où A⁴ représente l'atome d'hydrogène, le radical hydroxy, méthyle, éthyle, n- ou i-propyle, méthoxy, éthoxy, n- ou i-propoxy, méthylsulfonyle ou éthylsulfonyle,
A¹ représente par ailleurs, le radical méthylène, éthane-1,1-diyle, éthane-1,2-diyle, propane-1,1-diyle, propane-1,2-diyle, propane-1,3-diyle, éthène-1,2-diyle, propène-1,2-diyle, propène-1,3-diyle, éthyne-1,2-diyle, propyne-1,2-diyle ou propyne-1,3-diyle,
A² représente une simple liaison ou l'atome d'oxygène, de soufre, le radical -SO-, -SO₂-, -CO- ou le groupement -N-A⁴-, où A⁴ représente l'atome d'hydrogène, le radical hydroxy, méthyle, éthyle, n- ou i-propyle, méthoxy, éthoxy, n- ou i-propoxy, méthylsulfonyle, éthylsulfonyle, n- ou i-propylsulfonyle ou phénylsulfonyle,
A² représente par ailleurs, le radical méthylène, éthane-1,1-diyle, éthane-1,2-diyle, propane-1,1-diyle, propane-1,2-diyle, propane-1,3-diyle, éthène-1,2-diyle, propène-1,2-diyle, propène-1,3-diyle, éhyne-1,2-diyle, propyne-1,2-diyle ou propyne-1,3-diyle,
A³ représente l'atome d'hydrogène, avec la condition que dans ce cas, A¹ et/ou A² ne représentent pas une simple liaison,
A³ représente par ailleurs, le radical hydroxy, amino, cyano, nitro, carboxy, carbamoyle, sulfo, l'atome de fluor, de chlore, de brome,
A³ représente par ailleurs, le radical méthyle, éthyle, n- ou i-propyle, n-, i-, s- ou t-butyle, n-, i-, s- ou t-pentyle, méthoxy, éthoxy, n- ou i-propoxy, n-, i-, s- ou t-butoxy, n-, i-, s- ou t-pentoxy, méthylthio, éthylthio, n- ou i-propylthio, n-, i-, s- ou t-butylthio, méthylsulfonyle, éthylsulfonyle, n- ou i-propylsulfonyle, méthylamino, éthylamino, n- ou i-propylamino, n-, i-, s- ou t-butylamino, diméthylamino, diéthylamino, méthoxycarbonyle, éthoxycarbonyle, n- ou i-propoxycarbonyle, diméthoxyphosphoryle, diéthoxyphosphoryle ou dipropoxyphosphoryle, diisopropoxyphosphoryle, chaque fois le cas échéant substitué par l'atome de fluor, de chlore ou le radical méthoxy ou éthoxy,
A³ représente par ailleurs, le radical propényle, butényle, propényloxy, butényloxy, propénylamino, buténylamino, propylidèneamino, butylidèneamino, propényloxycarbonyle, butényloxycarbonyle, propynyle, butynyle, propynyloxy, butynyloxy, propynylamino, butynylamino, propynyloxycarbonyle ou butynyloxycarbonyle, chaque fois le cas échéant substitué par l'atome de fluor ou de chlore,
A³ représente par ailleurs, le radical cyclopropyle, cyclobutyle, cyclopentyle, cyclohexyle, cyclopropyloxy, cyclobutyloxy, cyclopentyloxy, cyclohexyloxy, cyclopropylméthyle, cyclobutylméthyle, cyclopentylméthyle, cyclohexylméthyle, cyclopropylméthoxy, cyclobutylméthoxy, cyclopentylméthoxy, cyclohexylméthoxy, cyclopentylidèneamino, cyclohexylidèneamino, cyclopentyloxycarbonyle, cyclohexyloxycarbonyle, cyclopentylméthoxycarbonyle ou cyclohexylméthoxycarbonyle, chaque fois le cas échéant substitué par l'atome de fluor, de chlore, le radical cyano, carboxy, méthyle, éthyle, n- ou i-propyle, méthoxycarbonyle ou éthoxycarbonyle,
A³ représente par ailleurs, le radical phényle, phényloxy, benzyle, phényléthyle, benzyloxy, phényloxycarbonyle, benzyloxycarbonyle, chaque fois le cas échéant substitué par le radical nitro, cyano, carboxy, l'atome de fluor, de chlore, de brome, le radical méthyle, éthyle, n- ou i-propyle, trifluorométhyle, méthoxy, éthoxy, n- ou i-propoxy, difluorométhoxy, trifluorométhoxy, méthoxycarbonyle et/ou éthoxycarbonyle,
A³ représente par ailleurs, un radical pyrrolyle, pyrazolyle, imidazolyle, triazolyle, furyle, thiényle, oxazolyle, isoxazolyle, thiazolyle, isothiazolyle, oxadiazoylyle, thiadiazolyle, pyridinyle, pyrimidinyle, triazinyle, pyrazolylméthyle, furylméthyle, thiénylméthyle, oxazolylméthyle, isoxazolylméthyle, thiazolylméthyle, pyridinylméthyle, pyrimidinylméthylé, pyrazolylméthoxy, furylméthoxy ou pyridylméthoxy, chaque fois le cas échéant complètement ou partiellement hydrogéné,
R⁷ représente l'atome d'hydrogène, de fluor ou de chlore.

3. Procédé de préparation de composés suivant la revendication 1 ou 2, **caractérisé en ce que**
(a) on condense par cyclisation, des dérivé (thio)semicarbazide de la formule générale (II) : 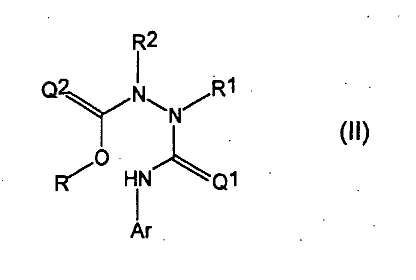 dans laquelle :
Q¹, Q², R¹, R² et Ar ont les significations données à la revendication 1, et
R représente un radical alcoyle,
le cas échéant en présence d'un auxiliaire de réaction et le cas échéant en présence d'un agent de dilution, et le cas échéant à la suite de cela, dans le cadre de la définition des substituants, on réalise des réactions de substitution électrophile ou nucléophile de manière usuelle,
ou **en ce que**
(b) on isomérise de manière thermique (« pyrolytique ») des aryliminohétérocycles de la formule générale (III) : 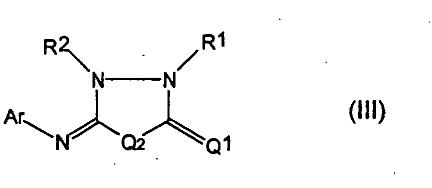
dans laquelle :
Q¹, Q², R¹, R² et Ar ont les significations données ci-dessus,
le cas échéant en présence d'un auxiliaire de réaction et le cas échéant, en présence d'un agent de dilution.

4. Agent herbicide, **caractérisé par** une teneur en au moins un composé selon la revendication 1 ou 2.

5. Procédé pour lutter contre les plantes non souhaitées, **caractérisé en ce que** l'on fait agir un composé selon la revendication 1 ou 2 sur les plantes non souhaitées et/ou sur leur biotope.

6. Utilisation d'un composé selon la revendication 1 ou 2, pour lutter contre les plantes non souhaitées.

7. Procédé de préparation d'agents herbicides, **caractérisé en ce que** l'on mélange un composé selon la revendication 1 ou 2 avec des agents de dilution et/ou des substances tensioactives.
